# EUROPEAN PATENT APPLICATION

(11) **EP 4 810 095 A2**
(43) Date of publication of application: **23.09.2026**
(21) Application number: 26176671.1
(22) Date of filing: 23.12.2020
(51) Int. Cl.: A61M 16/10

(54) **RESPIRATORY THERAPY SYSTEM, INCUBATOR, AND MEDICAL BREATHING GAS DELIVERY CONDUIT THEREFOR**

(30) Priority: 26.12.2019 US 201962953800 P
(62) Document number(s) of the earlier application(s) in accordance with Art. 76 EPC: 20904860.2 / 4 081 284
(71) Applicant: Fisher & Paykel Healthcare Limited, East Tamaki, Auckland 2013 (NZ)
(72) Inventor: BABBAGE, Sean Joel, Auckland 2013 (NZ); SANSON, Samuel Carey Mathew, Auckland 2013 (NZ)
(74) Representative: Haseltine Lake Kempner LLP

(57) **Abstract**

In accordance with this disclosure, we provide a medical conduit configured to deliver breathable gases in a respiratory therapy system. The medical conduit comprises:
i. a first conduit end connector configured to be connected to a user interface;
ii. a second conduit end connector configured to be connected to a heated inspiratory conduit;
iii. the medical conduit further comprising at least one portion intermediate the first and second conduit end connectors made from a breathable material;
iv. the medical conduit being configured to connect the user interface to the heated inspiratory conduit;
v. the medical conduit being configured, when connected to the user interface and the heated inspiratory conduit, to be located in an incubator; wherein
vi. the medical conduit is unheated.

Such a medical conduit can be used in a respiratory therapy system, which comprises an incubator, with the medical conduit inside the incubator.

## Description

### INCORPORATION BY REFERENCE

This application is related to international PCT applications having publication numbers WO2006/019323 filed 19 August 2005, WO2014/077706 filed 14 November 2013, WO2016/048172 filed 24 September 2015, WO2013/022356 filed 10 August 2012, WO2017037660 filed 2 September 2016, WO2011/077250 filed 22 December 2010, and WO2013/073970 filed 21 September 2012, each of which is incorporated herein by reference in its entirety.

### FIELD OF THE DISCLOSURE

The present disclosure generally relates to respiratory therapy systems for providing breathable gases to users, and more particularly to medical breathing gas delivery conduits in breathing circuits used with respiratory therapy systems that include humidifiers.

### BACKGROUND

Many respiratory therapy systems deliver breathable gases, and sometimes also heated and/or humidified, breathable gases for various medical procedures, including respiratory treatment, laparoscopy, and the like. These systems can be configured to control temperature, humidity and flow rates using feedback from one or more sensors. To maintain desirable properties of the breathable gases upon delivery to a user, a breathing circuit of the respiratory therapy system can have one or more heaters associated with components in the breathing circuit, such as gases conduits, where the one or more heaters provide heat to the breathable gases as it flows to the user. The conduit heater(s) can be controlled to provide heat to the breathable gases so that the breathable gases arrives to the user having desirable properties such as temperature and/or humidity. Such a respiratory therapy system can include a temperature sensor to provide feedback to a controller which can adjust and/or modify power delivered to the conduit heater(s) to achieve a target temperature at a location along an associated gases conduit.

An inspiratory circuit of a respiratory therapy system can be provided which comprises a heated conduit with a temperature sensor located at the user end of said conduit to measure the temperature of the gases flow being provided to the user. An example of such a circuit is described in our earlier PCT application WO2006/019323 (herein WO'323).

The main purpose of heating the inspiratory flow of gases occurs when the respiratory therapy system includes a humidifier (for example, as a standalone humidifier or as part of an integrated humidifier-flow generator). The humidifier heats and humidifies the respiratory gases to a target dewpoint, which are then delivered to the user via the inspiratory conduit. The inspiratory conduit is the term typically used to describe the gases conduit of the inspiratory circuit that is connected between the humidifier outlet and the user interface that is typically worn by the user. Without heating the inspiratory conduit, the temperature of these gases can then drop as they pass to the user interface. If the temperature of the gases drops below the dewpoint at any point in the inspiratory circuit, then condensation or "rainout" can occur.

The heated conduit described in WO'323 prevents condensation by heating the gases passing through the conduit. The power supplied to the conduit is regulated by one or more controllers of the system using a temperature signal provided by the temperature sensor located at the user end of the inspiratory conduit. The controller sets a target temperature for the user end of the inspiratory conduit, and then adjusts the power supplied to the conduit based on the difference between said target temperature and the measured value. The target temperature is generally set at or above the measured, estimated, or target dewpoint temperature of the gases exiting the humidifier. The heat produced by the heater (for example one or more heater wires) of the heated conduit is relatively consistent across the length of the conduit, and provided the rate of heat loss is also relatively consistent across the length of the conduit, then the temperature should be maintained above the dewpoint throughout the conduit.

### SUMMARY

The systems, methods and devices described herein have innovative aspects, no single one of which is indispensable or solely responsible for their desirable attributes. Without limiting the scope of the claims, some of the advantageous features will now be summarized.

Throughout, the term 'respiratory therapy system' can be interchanged with 'breathing assistance apparatus'.

Throughout, the term "circuit" in this specification means the entire breathable gases inspiratory pathway to the user from a gases supply, and may also include the expiratory gases path away from the user to a gases supply. The circuit at a minimum should therefore include the inspiratory gases pathway (including all the components) from the gases supply to the user interface. The interface itself e.g. mask or cannula, is separate from the gases pathway and not part of the 'circuit'.

Throughout, 'gases conduit' is any conduit configured to transport breathable gases or respiratory gases.

Some embodiments provide for an inspiratory limb for a breathing circuit. The inspiratory limb described herein is particularly useful in situations where heated and humidified gases must pass through two distinct environments. This can be a problem, for example, in infant incubators where the temperature may be significantly higher than the surrounding environment or where a portion of the conduit delivering the gases to the user is under a blanket. The embodiments disclosed herein, however, can be used in any environment where heated and/or humidified gas is delivered to a user where the inspiratory limb passes through two distinct environments having one or more different conditions.

According to an aspect of this disclosure there is provided a medical conduit configured to deliver breathable gases in a respiratory therapy system; the medical conduit comprising:
a first conduit end connector configured to be connected to a user interface;
a second conduit end connector configured to be connected to a heated inspiratory conduit;
the medical conduit further comprising at least one portion intermediate the first and second conduit end connectors made from a breathable material;
the medical conduit being configured to connect the user interface to the heated inspiratory conduit;
the medical conduit being configured, when connected to the user interface and the heated inspiratory conduit, to be located in an incubator.

According to an aspect of this disclosure there is provided a medical conduit configured to deliver breathable gases in a respiratory therapy system; the medical conduit comprising:
a first conduit end connector configured to be connected to a user interface;
a second conduit end connector configured to be connected to a heated inspiratory conduit;
the medical conduit further comprising at least one portion intermediate the first and second conduit end connectors made from a breathable material;
the medical conduit being configured to connect the user interface to the heated inspiratory conduit;
the medical conduit being configured, when connected to the user interface and the heated inspiratory conduit, to be located in an incubator; wherein
the medical conduit is unheated.

According to an aspect of this disclosure there is provided an incubator breathing gas delivery conduit configured to deliver breathable gases in a respiratory therapy system including an incubator; the medical conduit comprising:
a first conduit end connector configured to be connected to a user interface inside the incubator;
a second conduit end connector configured to be connected to a heated inspiratory conduit outside the incubator;
the incubator breathing gas delivery conduit further comprising at least one portion intermediate the first and second conduit end connectors made from a breathable material.

The conduit may be unheated, that is, the medical conduit does not comprise any heater to heat gases flowing through the medical conduit.

The conduit may comprise an elongate film spirally wrapped with an elongate reinforcing member to form a lumen, the elongate film bonding with the elongate reinforcing member.

The conduit may comprise one or more thermally insulating portions, for example formed by one or more air pockets or other insulating material.

The at least one portion of the medical conduit may be made from breathable material comprises at least one portion of the elongate film.

The conduit may be compressible, that is, the length of the conduit can be reduced.

The conduit may be extensible, that is, the length of the conduit can be increased.

The conduit may be breathable in that at least part of the conduit is highly permeable to moisture vapor such as water vapor, but is substantially impermeable to liquid moisture such as liquid water and substantially impermeable to the bulk flow of gases.

The length of the conduit may be between about 20cm and 35cm, and in some configurations is about 25cm. The length of the conduit may be between 10cm and 50cm, in some configurations between 15cm and 40cm, and in some configurations between 20cm and 35 cm.

The length of the conduit may be less than the length of the heated inspiratory conduit with which the conduit is used.

The conduit of any one of the preceding claims may define a lumen through the conduit and along which breathable gases flow, the lumen comprising a diameter, the diameter being in the range of 3mm to 40mm, preferably 5mm to 25mm and more preferably 10mm to 20mm.

The breathable material is advantageous as it helps to manage condensation within the conduit. Condensation can occur due to excess water vapor in the gases passing through the conduit. The breathable material allows transmission of water vapor out of the conduit walls while reducing or preventing transmission of liquid water out of the conduit. The breathable material may be further configured to absorb liquid water into the wall of the conduit. The conduit in the incubator made of breathable material is advantageous because it reduces condensate in the conduit.

According to an aspect of this disclosure there is provided a respiratory therapy kit for use in a respiratory therapy system, the kit comprising:
a conduit according to any one of the above statements;
a user interface configured to be secured to a user's head to deliver breathable gases to the user; and
a heated inspiratory conduit configured to receive humidified breathable gases from a humidifier of the respiratory therapy system.

The user interface may comprise an interface conduit.

The conduit may comprise:
a first conduit end connector configured to be connected to a connector of the user interface, and a second conduit end connector configured to be connected to an end connector of the heated inspiratory conduit; wherein
the first conduit end connector is also configured to be connectable to the second conduit end connector. The conduit comprises a breathable material.

The conduit may comprise:
a first conduit end connector configured to be connected to a connector of the user interface, and a second conduit end connector configured to be connected to an end connector of the heated inspiratory conduit; wherein
the end connector of the heated inspiratory conduit is configured to be connected directly to the connector of the user interface, without using the medical conduit.

One or both ends of a conduit according to any one of the above statements may comprise connector features for releasably connecting the conduit to another component of a breathing circuit. The connector features may be configured such that the conduit can only be connected in a given orientation in the breathing circuit.

One end of the conduit may comprise:
a first conduit body; and
one or more locking fingers protruding longitudinally from the conduit body.

The other end of the conduit may comprise:
a second conduit body; and
one or more locking tabs configured to releasably attach with the one or more locking fingers of the first conduit body, the one or more locking tabs formed on an interior surface of the second conduit body.

The first or second conduit end connector of the conduit may comprise the first conduit body. The other of the first or second conduit end connector of the conduit may comprise the second conduit body.

The first conduit end connector may comprise a sensor port for receiving a sensor probe, the sensor port formed substantially perpendicularly adjacent to an aperture on the second conduit body, the aperture configured to receive the connecting adapter into the second conduit body.

The sensor port may form an opening into an insertion aperture formed by the one or more locking fingers when attached to the first conduit connector, the opening configured to allow the sensor probe to extend into the insertion aperture.

The sensor port may be formed on the second conduit body such that the ends of the one or more locking fingers extend past the opening formed by the sensor port when attached to the first conduit connector.

The first conduit end connector may comprise a sensor probe positioned to fit between an insertion aperture formed by the one or more locking fingers.

The first conduit end connector may comprise a receptacle for receiving a detachable sensor probe, the receptacle formed within the second conduit body, the receptacle configured to position the detachable sensor probe between an insertion aperture formed by the one or more locking fingers.

Full engagement of the one or more locking tabs with the one or more locking fingers may generate an audible sound.

The first and second conduit end connectors may be configured to provide a quick connect mechanism between the first conduit end connector and the second conduit end connector.

An alignment tab may be formed on an interior surface of the second conduit body, the alignment tab configured to automatically align the one or more locking fingers with the one or more locking tabs upon insertion of the second conduit end connector within the first conduit end connector.

The one or more locking fingers may comprise two locking fingers.

In an incubator breathing circuit, comprising a conduit according to any one of the above statements, a user interface inside the incubator, and a heated inspiratory conduit outside the incubator, each of the conduit, user interface and heated inspiratory conduit may comprise:
a first conduit body having one or more locking fingers protruding longitudinally from the conduit body; and/or
a second conduit body having one or more locking tabs configured to releasably attach with the one or more locking fingers of the first conduit body, the one or more locking tabs formed on an interior surface of the second conduit body.

Each of the conduit, user interface, and heated inspiratory conduit comprises connector features that enable each component to be releasably connected to the next. The connector features can be arranged so that each component can only be connected to the appropriate adjacent component, or can be arranged so that each component can be selectively connected to any one or more of the other components.

In a further aspect of this disclosure, there is provided a connector to be provided at a terminal end of a medical conduit according to any one of the above statements, the connector comprising:
a body, the body comprising a first end and a second end, the body internally defining a lumen for the passage of gas therethrough between each of the first and second ends, the first end, in use, being engaged or engageable with the terminal end of the medical conduit or at least a component to be associated with the terminal end of the medical conduit, and
the second end, in use, to be engaged or engageable with another connector, and
wherein an internal surface of the body comprises one or more internal connection features configured for connection with said another connector which may be received internally therein, and
wherein an external surface of the body comprises one or more external alignment feature(s) configured for aligning said connector or another connection into an externally aligned connection therebetween.

In another aspect of this disclosure, there is provided a connector to be provided at a terminal end of a medical conduit according to any one of the above statements, the connector comprising:
a body, the body comprising a first end and a second end, the body internally defining a lumen for the passage of gas therethrough between each of the first and second ends, the first end, in use, being engaged or engageable with the terminal end of the breathing conduit or at least a component to be associated with the terminal end of the breathing tube, and
the second end, in use, to be engaged or engageable with another connector, and
wherein an internal surface of the body comprises one or more internal connection features configured for connection with said another connector which may be received internally therein, and
wherein one or more external visual aid(s) is/are configured for, in use, providing an externally visible guide for alignment of said connector or another connector into an aligned connection therebetween.

According to the above two aspects, there is provided one or more additional features described by the optional configurations below.

The one or more internal connection features may be surface feature(s) that extend radially inward from the surface of an internal side wall of the body.

The one or more internal connection features may comprise one or more tabs.

The one or more tab(s) may be a raised protrusion.

The internal connection features may be oriented so as to be radially aligned with said one or more external alignment features and/or said one or more external visual aids.

There may be a pair of said internal connection features.

At least one (optionally only one) of said internal connection features may comprise a longitudinally extensive channel or recess, said channel or recess may be configured to locate, retain, or position a printed circuit board (PCB) arrangement.

The internal surface may comprise one or more internal alignment features configured for aligning at least one connection feature of another connector to be received internally thereof into an aligned connection orientation therewith.

Said internal surface may comprise one or more internal alignment features configured to, in use, rotatably orient a male connection feature of another connector into an aligned connection orientation for connection with the connector, or at least into connection with, said one or more internal connection features located on or about the internal surface of the body.

The internal alignment feature(s) may be surface feature(s) that extend radially inward from the surface of an internal side wall of the body.

The internal alignment feature(s) may comprise one or more tab(s).

The one or more tab(s) may be a raised protrusion.

The one or more tab(s) may comprise a pair of shoulders, sloping away from each other and away from an end of the tab at the intersection of the shoulders, the end of the tab located substantially more toward a terminal end of the second end of the connector than the shoulders.

The internal alignment feature(s) may be one or more ribs extending substantially in a longitudinal direction of said connector and along said internal surface, optionally being a surface of an internal side wall of the body.

The internal alignment features may comprise: 1-10 ribs, or 2-8 ribs, or 4-6 ribs, or 2 ribs, or 3 ribs, or 4 ribs, or 6 ribs, or 8 ribs, or 10 ribs.

Two or more sets of internal alignment features may be provided on or about the internal surface of the body, optionally there are two sets of alignment features.

Each said set of internal alignment may comprise an equal number of internal alignment features as another set.

The internal surface may comprise two of said internal alignment features.

The internal alignment features may, in use, rotatably align a pair of fingers extending from another connector when inserted into or placed into engagement or surface contact with said internal surface of the connector.

The first end may be configured for engagement with the terminal end of the breathing conduit.

The first end may comprise a sleeved portion to be attached to the terminal end of the breathing conduit and to form a pneumatic connection therewith.

At least a part of said sleeved portion may be insertable into or to be located or housed within an interior surface or the lumen of the terminal end of the breathing conduit.

At least a part of said sleeved portion may be receivable upon or to be located or housed upon an exterior surface of the terminal end of the breathing conduit.

The external alignment feature(s) and/or the external visual aid(s) may comprise one or more external surface features extending radially outwardly from the outer surface of an external side wall of the body.

The external alignment feature(s) may comprise one or more tab(s).

The one or more tab(s) may be a raised protrusion.

The external alignment feature(s) may be one or more rib(s) (or protrusion(s)) extending substantially in a longitudinal direction with the connector and along said external surface, optionally being a surface of an external side wall of the body.

The at least one, or each rib or protrusion may comprise a pair of shoulders, said shoulders sloping away from each other and away from an end of the rib or protrusion at the intersection of the shoulders, the end of the rib or protrusion located substantially more toward a terminal end of the second end of the connector than the shoulders.

The at least one, or each rib or protrusion may be substantially tongue shaped, and/or substantially triangular and/or substantially tapers toward an end.

The external alignment features may comprise: 1-10 ribs, or 2-8 ribs, or 4-6 ribs, or 2 ribs, or 3 ribs, or 4 ribs, or 6 ribs, or 8 ribs, or 10 ribs.

External alignment features may be spaced, arrayed or arranged evenly or equidistantly from each other about the circumference or a radius of the external surface.

The external alignment feature may be a projection of a length that extends in a substantially longitudinal direction of said connector and along said external surface, and a height of said projection from the external surface varies along said length.

The height of said external alignment feature may taper along said length.

The height of said projection may either:
a. reduce in a direction extending from a base of the external alignment feature toward a terminal end of the second end of the connector, or
b. increase in a direction extending from a base of the external alignment feature toward a terminal end of the second end of the connector.

Provided substantially at or toward a base of a or each said external alignment feature may be a stepped protrusion, the stepped protrusion being a more radially outwardly extending projection than an adjacent portion of the external alignment feature.

The stepped projection may be configured to be co-located or co-locatable for keying with a reciprocally shaped recess or cut-out of at least a part of a sleeved portion of another connector when brought to bear into connection therewith during a connection between the connector and said another connector.

The stepped projection may be configured to act as a key to
reciprocally locate with a reciprocally shaped recess or cut-out of a component brought into connection therewith.

Provided substantially at or toward a base of a or each said external alignment feature may be a recess or cut-out, the recess or cut-out configured for receiving a protrusion or projection of a reciprocally sha ped portion of another connector.

The recess or cut-out may be configured to be co-located or co-locatable for keying with a reciprocal protrusion or projection of at least a part of a sleeved portion of another connector when brought to bear into connection therewith during a connection between the connector and said another connector.

The recess or cut-out may be configured to act as a keyway to reciprocally locate with a reciprocally shaped protrusion or projection of a component brought into connection therewith.

The stepped protrusion or recess or cut-out may be of the following shapes or profiles for locating with or receiving a substantially reciprocally shaped recess or cut-out, or a protrusion or projection: semi-circular, triangular, rectangular or other recti-linear or geometric shapes, elliptical, wedge shaped.

A radially extensive flange or lip may project from the external surface of the body.

Said flange or lip may substantially defines a stop end for a point or length of maximum engagement of another connector when made with the external surface of the connector.

Said flange or lip may comprise one or both of:
a. one or more radially and/or longitudinally recessed or grooved regions, or
b. one or more radially and/or longitudinally extending projection regions.

Said flange or lip may be longitudinally extensive so as to be configured for an engagement with the terminal end of the medical conduit.

According to an aspect of this disclosure there is provided a respiratory therapy kit for use in a respiratory therapy system, the kit comprising:
the conduit of any one of the above statements; and
a heated inspiratory conduit configured to receive humidified breathable gases from a humidifier of the respiratory therapy system;
the medical conduit comprising:
   a first conduit end connector configured to be connected to a connector of a user interface of the respiratory therapy system, and a second conduit end connector configured to be connected to an end connector of the heated inspiratory conduit;
wherein
the first conduit end connector is also configured to be connectable to the second conduit end connector.

According to an aspect of this disclosure there is provided a respiratory therapy kit for use in a respiratory therapy system, the kit comprising:
the conduit of any one of the above statements; and
a heated inspiratory conduit configured to receive humidified breathable gases from a humidifier of the respiratory therapy system;
the medical conduit comprising:
   a first conduit end connector configured to be connected to a connector of a user interface of the respiratory therapy system, and a second conduit end connector configured to be connected to an end connector of the heated inspiratory conduit;
wherein
the end connector of the heated inspiratory conduit is configured to be connected directly to a connector of the user interface, without using the medical conduit.

The heated inspiratory conduit may comprise a temperature sensor, configured to measure the temperature of breathable gases in the heated inspiratory conduit.

The connector on the user interface may be substantially identical to the second conduit end connector of the conduit.

The kit of any one of the above statements may comprise a plurality of incubator gases delivery conduits, wherein at least one incubator gases delivery conduit comprises a diameter of 5mm, at least a second incubator delivery conduit comprises a diameter of 10mm, and at least a third incubator delivery conduit comprises a diameter of 15mm. Additional incubator delivery conduits can be provided with additional diameters. In use, an incubator gases delivery conduit may be selected depending on the size of the user interface, wherein the selected incubator gases delivery conduit diameter corresponds to the diameter of an interface conduit of the user interface.

An incubator comprising the conduit of any one of the above statements; and/or a respiratory therapy kit of any one of the above statements.

The heated inspiratory conduit may comprise a temperature sensor, configured to measure the temperature of breathable gases in the heated inspiratory conduit.

The heated inspiratory conduit may comprise a controller, wherein the controller uses an output of the temperature sensor to control the heat delivered to the breathable gases by the heated inspiratory conduit, the controller controlling the heat delivered based on the dewpoint of the breathable gases.

The temperature sensor may be positioned outside of the incubator.

The conduit and the heated inspiratory conduit are may be configured such that the conduit is inside the incubator, and the heated inspiratory conduit is outside of the incubator.

The second conduit end connector of the conduit may be positioned outside the incubator, when connected to the heated gases conduit.

The second conduit end connector of the conduit may be positioned inside the incubator, when connected to the heated gases conduit.

The incubator may comprise an incubation enclosure in which the user is contained in use, and the medical conduit has a length, the length being sufficient to extend from a periphery of the incubation enclosure to the centre of the incubation enclosure.

According to an aspect of this disclosure there is provided a respiratory therapy system comprising:
a flow generator;
a humidifier;
a heated inspiratory conduit;
a user interface coupled to the gases conduit to deliver a gases flow to a user;
a sensor configured to determine pressure or flow of the gases flow;
a controller configured to control the flow generator to generate the gases flow;
the system further comprising the conduit and/or the respiratory therapy kit of any one of the above statements.

The heated inspiratory conduit may comprise a temperature sensor, configured to measure the temperature of breathable gases in the heated inspiratory conduit.

The controller may use an output of the temperature sensor to control the heat delivered to the breathable gases by the heated inspiratory conduit, the controller controlling the heat delivered based on the dewpoint of the breathable gases.

The respiratory therapy system may be configured for use in an environment comprising both an ambient environment, subject to ambient environment conditions, and a controlled environment subject to at least one controlled parameter, wherein the gases conduit medical conduit is configured to be located in the ambient environment, and the medical conduit is configured, when connected to the gases conduit, to be located in the controlled environment.

The controlled parameter may be selected from any of:
a. temperature;
b. humidity;
c. pressure;
d. flow

The user interface may be any one of:
a. a nasal interface configured to seal around the nares or nose of the user;
b. an oral mask configured to seal around the mouth of the user;
c. a full face mask configured to seal around both the mouth and nose of the user;
d. a nasal mask, which may be an infant nasal mask; or
e. an unsealed nasal cannula.

The respiratory therapy system may comprise any one or more further components being:
a. an elbow connector configured to connect a gases conduit to a user interface;
b. a gases conduit;
c. a gases conduit connector configured to connect a gases conduit to another component of the respiratory therapy system.

The respiratory therapy system may comprise any one or more of:
a. a breathing gas flow generator;
b. a b humidifier for humidifying the breathable gases;
c. a gases conduit, which may or may not be heated; and/or
d. a user interface;
e. a supplementary gases inlet to allow supplementary gases to be introduced into the respiratory therapy system.

The supplementary gases are preferably delivered upstream of the flow generator. The flow generator may be used to mix the supplementary gases and ambient air or ambient gases that are drawn in through an inlet of the respiratory therapy system by the flow generator. Alternatively the respiratory therapy system may comprise a mixer to mix the supplementary gases and ambient air.

The respiratory system may comprise a first supplementary gases inlet and a second supplementary gases inlet. The first supplementary gases inlet is a low pressure oxygen inlet. The second supplementary gases inlet may comprise a high pressure gases inlet. A valve may be coupled to the second supplementary gases inlet to control the amount of supplementary gases introduced into the gases flow. The respiratory gases supply comprises a controller to control the flow generator, humidifier and the valve to control the amount of supplementary gases. In one example the valve is controlled to control the oxygen fraction in the gases stream.

The respiratory gases supply may also comprise one or more sensors that are attached to a patient to measure a patient parameter e.g. the patient's SpO2 readings. In one example the sensor attached to the patient may be a pulse oximeter that is used to measure the patient's SpO2. The controller may be configured to control the flow generator to deliver a required or set flow rate. The controller may further be configured to control the valve to maintain an oxygen fraction that achieves a set SpO2 value. The controller is configured to execute a closed loop SpO2 control scheme that attempts to maintain a patient's SpO2 value by controlling the oxygen fraction within the gases stream.

The breathable material of the medical conduit may be configured to manage condensation within the conduit, the breathable material allowing transmission of water vapor out of the conduit while reducing or preventing transmission of liquid water out of the conduit.

The breathable material may be configured to absorb liquid water into the conduit.

The breathable material may reduce condensate in the conduit.

When using a medical conduit according to any of the above statements, it can be important that the unheated medical conduit remain inside the incubator, and the heated inspiratory conduit remain outside of the incubator. We have determined that it can therefore be desirable for the connector, connecting the outlet end of the heated inspiratory conduit to the inlet end of the unheated medical conduit to be secured at the boundary of the incubator. To achieve this, once the heated inspiratory tube is located in the correct position, a clip can be provided on the heated inspiratory tube, to clip the heated inspiratory tube to the exterior of the incubator, to resist movement of the heated inspiratory conduit into the incubator, and/or to resist movement of the unheated medical conduit from the incubator. Once so secured, movement of the patient will be less likely to alter the position of the connector.

According to a further aspect of this disclosure, we provide a component for use with the conduit, kit, and/or respiratory therapy system of any one or more of the above statements, the component comprising a body engageable with one or more external surface recesses of respective one or more conduits, and a pair of jaws extending from the body for gripping an item such that, in use, in a first orientation of the body relative to the respective conduit(s) recesses, the component is movable along a length of the conduit(s), and in a second orientation of the body relative to the respective conduit(s) recesses, the component is resistive to movement along a length of the conduit(s).

Preferably in use, the body is substantially surrounding of a perimeter of the conduit(s).

Preferably an internal surface of the body is engageable with the one or more external surface recesses of the conduit(s).

Preferably in the first orientation the component is in a plane such that the body engageable with external recesses of the respective conduit(s) is substantially co-axial with the respective conduit(s), and in the second orientation the component is in a plane such that the body is engageable with external recesses of the respective conduit(s) is substantially non co-axial with the respective conduit(s).

Preferably one or more portions extend from the body to be engageable with the recesses of the conduit(s).

Preferably the one or more portions is/are fixed portions.

Preferably the one or more portions is/are an annular lip or a projection or projections extending from an internal surface of the body.

Preferably the jaws are opposing upon one another in a closed position. Preferably the jaws are co-acting upon each other in a closed position.

Preferably the jaws are hingedly biased toward each other in reaching a substantially closed position.

Preferably the jaws are openable from a substantially closed position for gripping of an item, openable by deflecting the jaws away from each other.

Preferably the jaws are openable and grippable of an item for locating of the conduit engaged by the component in a set position.

Preferably the component is positioned on the conduit(s), such that, when the component is in the second orientation, and the jaws are gripping of an item, the component acts to locate the conduit(s) in a set position.

Preferably the item comprises one or more of the following: clothing, bedding, structures associated with personal clothing (e.g. personal lanyard, belt) or bedding (e.g. bed frame, mattress), structures associated with medical equipment or where a user is located (e.g. stands, bed side table), incubators, or cots.

Preferably the body comprises a shoulder portion associated with each jaw of the pair of jaws, the shoulder portion providing a surface for actuation, by a user, of the jaws to an open position.

Preferably the shoulder portion is an enlarged region of the body.

Preferably in use, the shoulder portions are deflectable towards each other, such that, in-use, deflection moves the jaws from a closed or substantially closed position to or toward a substantially open position, and release of the deflection allows the hingedly biased jaws to move back to the closed or substantially closed position.

Preferably the shoulder portions are sized for actuation by fingers of a user, or are finger tabs.

Preferably one or each jaw comprises grips for gripping of an item.

Preferably the grips are one or a series of ridges, projections or teeth, such grips being interlockable or interposing with one or more corresponding grips of an opposing jaw.

Preferably one of more of the grips is shaped to expose one or a series of acute angled portions facing inwardly toward the body.

Preferably one of more of the grips is shaped to expose one or a series of obtusely angled portions facing outwardly away from the body.

Preferably the body is configured to be substantially annular about the exterior surface of the, or each, respective conduit(s).

The system and kit as described earlier may include an expiratory conduit that is coupled to the patient interface and direct exhaled gases away from the patient. The expiratory conduit may direct and carry gases away and out of the incubator. The expiratory conduit may also comprise a breathable material. The breathable material may be similar to the breathable material of the medical conduit that is used between the interface conduit and the inspiratory conduit. The expiratory conduit being made of breathable material allows excess water vapour to be transferred out of the expiratory conduit to reduce or prevent condensation within the expiratory conduit.

### BRIEF DESCRIPTION OF THE DRAWINGS

Throughout the drawings, reference numbers can be reused to indicate general correspondence between reference elements. The drawings are provided to illustrate example embodiments described herein and are not intended to limit the scope of the disclosure.
**Figure 1** shows an example prior art respiratory humidification system for delivering humidified breathable gases to a user, the respiratory therapy system having a breathing circuit that includes an inspiratory circuit comprising a heated inspiratory conduit with a sensor at an end connector of the conduit.
**Figure 2** shows a respiratory therapy system in accordance with an aspect of this disclosure.
**Figure 3** is a first conduit end connector of a medical extension conduit of the system of Figure 2.
**Figure 4** is a second conduit end connector of a medical extension conduit of the system of Figure 2.
**Figure 5** shows an elastomeric outer cover configured for use with the second conduit end connector of Figure 4.
**Figure 6** shows the outer cover of Figure 5 in an assembled configuration with the second conduit end connector of Figure 4.

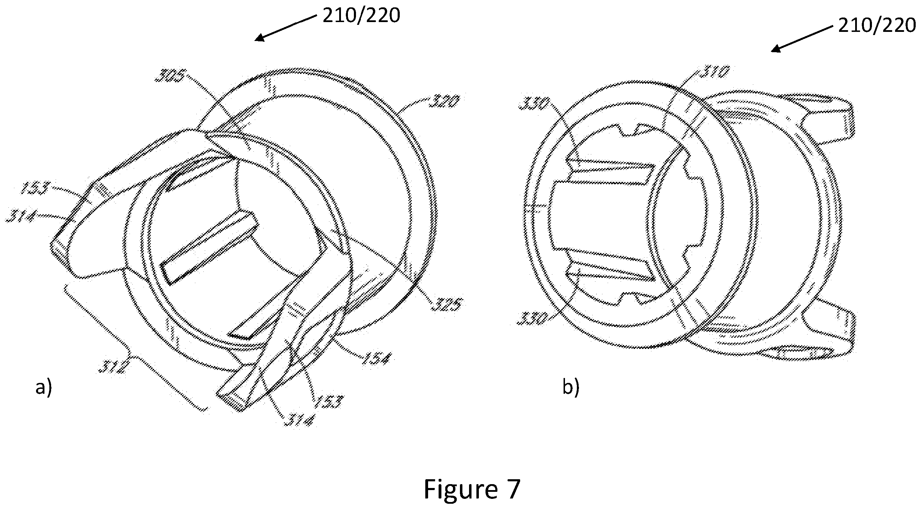

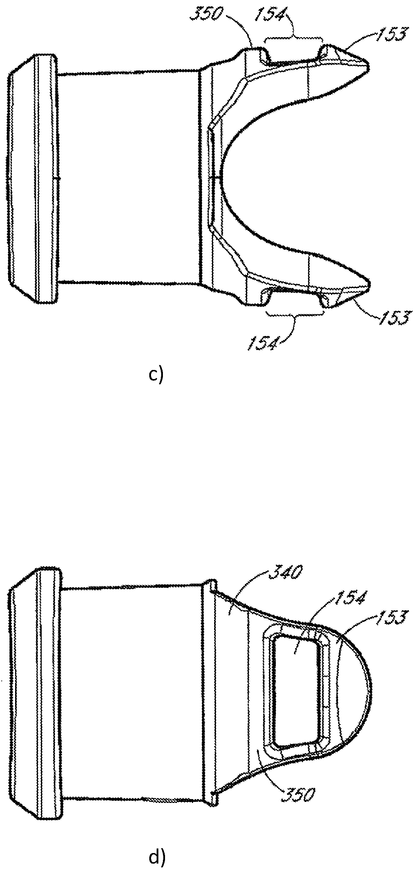

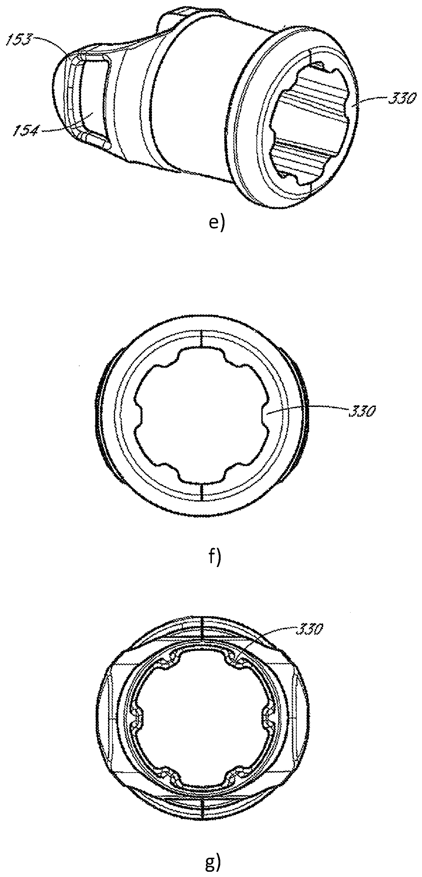

**Figures 7a to 7e** are various views of connection features of conduit connectors in accordance with this disclosure.
**Figure 8** is a perspective cross sectional view of an example medical extension conduit in accordance with this disclosure.
**Figure 9a** is a side-plan view of a medical extension conduit incorporating a breathable foamed polymer material; and **Figure 9b** is cross section view of the medical extension conduit of Figure 9a.
**Figure 10a** shows a side-plan view of a section of an example conduit.
**Figure 10b** shows a longitudinal cross-section of a top portion a conduit similar to the example conduit of Figure 10a.
**Figure 10c** shows another longitudinal cross-section illustrating a first elongate member in a conduit.
**Figure 10d** shows another longitudinal cross-section of a top portion of a conduit.
**Figure 10e** shows another longitudinal cross-section of a top portion of a conduit.
**Figure 11** shows a transverse cross-section of a second elongate member in a conduit.

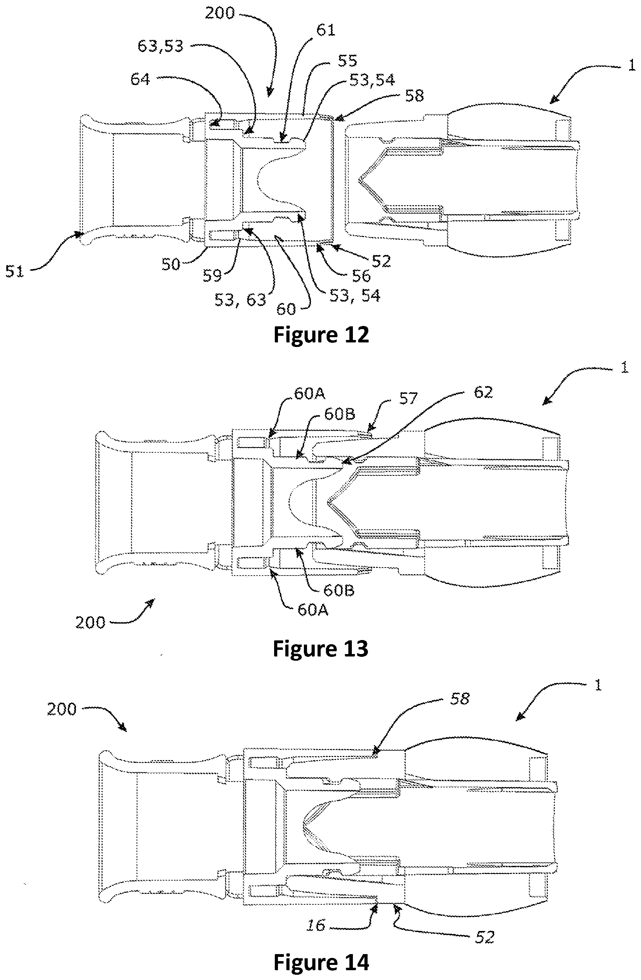

**Figures 12-14** illustrate a sequence in which the connector is connected or engaged with another connector.
**Figure 15** is a cross-section through the left-hand end connector of the connector arrangements shown in Figures 12-14.
**Figure 16** shows a graph of the humidity drop against flow rate for various setups of respiratory therapy system.
**Figure 17** shows a graph of the temperature drop against flow rate for various setups of respiratory therapy system.
**Figure 18** **is** a perspective view of a clip component in use with a conduit in accordance with this disclosure.
**Figure 19** is a side view corresponding to Figure 18.

### DETAILED DESCRIPTION

Certain embodiments and examples of a respiratory therapy system, such a system comprising or for use with an incubator, and medical conduits for such a system, are described herein. Those of skill in the art will appreciate that the disclosure extends beyond the specifically disclosed embodiments and/or uses and obvious modifications and equivalents thereof. Thus, it is intended that the scope of the disclosure herein disclosed should not be limited by any particular embodiments described herein.

The disclosure references heater wires, heating elements, and/or heaters in the context of providing heat to a conduit. Heater wire, for example, is a broad term and is to be given its ordinary and customary meaning to a person of ordinary skill in the art (that is, it is not to be limited to a special or customized meaning) and includes, without limitation, heater strips and/or conductive elements that produce heat when electrical power is provided. Examples of such heating elements include wires made of a conductive metal (*e.g.*, copper), conductive polymers, conductive inks printed on a surface of a conduit, conductive materials used to create a track on a conduit, and the like. Furthermore, the disclosure references conduits, limbs, and medical conduits in the context of gas delivery. Conduit, for example, is a broad term and is to be given its ordinary and customary meaning to a person of ordinary skill in the art and includes, without limitation, passageways having a variety of cross-sections such as cylindrical and non-cylindrical passageways. The disclosed systems and medical conduits can also be used in breathing circuits configured to provide a continuous, variable, or bilevel positive airway pressure (PAP) therapy or other form of respiratory therapy such as high flow or low flow oxygen therapy. The breathing circuit may for example comprising an inspiratory circuit which at a minimum includes the inspiratory gases pathway (including all the components) from the gases supply to the user interface.

When a heated, humidified breathing or gases conduit is used for an incubator or any other temperature (or other parameter) controlled environment (or any region where there is a temperature change, such as around radiant warmers used for burn victims, or under a blanket used by a user), the gases conduit may pass through at least two distinct zones: a lower temperature zone (such as the one outside the incubator, e.g. in ambient air) and a higher temperature zone (such as the one inside the incubator). If the gases conduit is heated by a single heater along its full length, one of the zones will tend to be at an undesirable, unsuitable, or non-optimal temperature, depending on which zone is sensed (*e.g.*, which zone contains a temperature sensor). If the heater is controlled to a sensor inside the incubator (such as to a user-end temperature sensor), the section outside the incubator will tend to be too cool, which can lead to condensation in the gases conduit.

Conversely, if the heater is controlled to a sensor outside the incubator, the section inside the incubator will tend to be too hot, which can lead to overheated gas being provided to the user. This can be particularly undesirable when providing respiratory therapy to infants, such as neonates,, where any excess temperature in the breathing gases delivered to the infant must be avoided. The overheating of the gases can also raise the enthalpy of the gases above a safe limit. This over enthalpy condition should be avoided.

In a situation where the temperature sensor is outside the incubator, and the temperature of the gases are not accurately controlled, the gases may cool across the conduit portion inside the incubator. This cooling can result in condensation of the water vapour in the gases. This condensation is a risk to a user e.g. an infant within the incubator. The liquid water that has condensed can present a drowning risk and may also present an infection risk, if the liquid water reaches the user. Further the liquid water provides a restriction within the conduit, thereby partially restricting or restricting gases flow through the conduit. This can reduce the effectiveness of the therapy delivered to the patient.

A typical example of an inspiratory circuit (of a breathing circuit which may or may not also comprise an expiratory circuit) in a respiratory therapy system, includes a heated gases conduit with a temperature sensor located at the user end of said conduit to measure the temperature of the gases flow being provided to the user. An example of such a circuit is described in our earlier patent application PCT publication WO2006/019323 (herein WO'323). Another example of such a circuit is described in our earlier patent application PCT publication WO2014/077706.

The respiratory therapy system may include a humidifier (for example, as a standalone humidifier or as part of an integrated humidifier-flow generator). The humidifier heats and humidifies the respiratory gases to a target dewpoint, which are then delivered to the user via the gases conduit. Without heating the gases conduit, the temperature of these gases can then drop as they pass to the user interface. If the temperature of the gases drops below the dewpoint at any point in the inspiratory circuit, then condensation or "rainout" can occur.

The heated conduit described in WO'323 prevents condensation by heating the gases passing through the conduit. The power supplied to the conduit is regulated by a controller in the device through reference to a temperature signal provided by the temperature sensor located at the user end of the inspiratory conduit. The controller sets a target temperature for the user end of the inspiratory conduit, and then adjusts the power supplied to the conduit based on the difference between said target temperature and the measured value. The target temperature is generally set at or above the measured, estimated, or target dewpoint temperature of the gases exiting the humidifier. The heat produced by the heater wire of the heated conduit is relatively consistent across the length of the conduit, and provided the rate of heat loss is also relatively consistent across the length of the conduit, then the temperature should be maintained above the dewpoint throughout the conduit.

When receiving respiratory support, infant users, such as neonatal users, are often placed in an incubator, which maintains the environment around the user at a fixed temperature. This environment is typically warmer than the room air (typically incubators are maintained between about 35°C and 37°C), and as such the ambient conditions surrounding the flow path from the device to the user interface become inconsistent along the length of the flow path (i.e. along the length of the inspiratory circuit). If the heated conduit described above was to be connected to user interface inside the incubator, then the user end of the heated conduit comprising the temperature sensor would also be located inside the heated incubator. This would cause the user end of the heated conduit and the temperature sensor to be heated by the incubator itself, resulting in a lower power being required to achieve the target temperature at the temperature sensor.

Whilst this lower power setting may be enough to achieve the target temperature at the temperature sensor, it might not be enough to maintain the temperature of the gases flow above the dewpoint temperature in the section of the conduit that is located outside of the incubator (i.e. the part of the conduit exposed to ambient environmental conditions). As such, there is a chance of condensation occurring if the circuit is set up in this way. This condensation can be a problem due to lack of sanitation, restricting or blocking the conduit, and/or posing a risk to the user should the liquid be tipped towards the interface. As such, it is desirable to be able to prevent or at least reduce this condensation, for example to help reduce the risk of drowning or infection

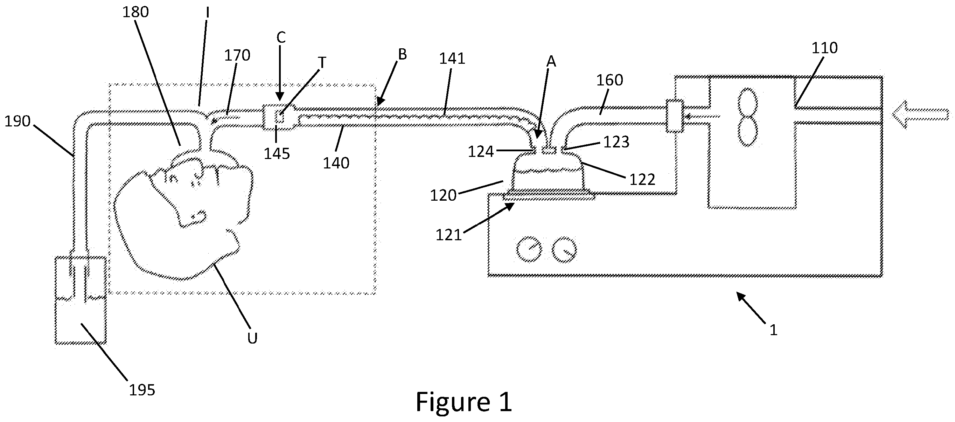

Figure 1 shows an example of a typical prior art respiratory therapy system 1 having a gases source 110 that is either integrated with, or a separate component from, a humidifier 120 comprising a humidifier heater plater 121 and a humidifier fluid chamber 122 having an inlet 123 and an outlet 124. The respiratory therapy system may comprise a housing comprising a casing or enclosure. The gases source 110, such as a blower, and the humidifier 120, may be integrated into the housing. The gases source 110 and humidifier 120 supplies heated and humidified breathable gases to a user U via a breathing circuit that includes, for example, an inspiratory gases conduit 140 for delivery of breathable gases, and a user interface 150. The gases conduit 140 comprises a conduit heater wire 141. In some embodiments, another medical conduit, such as a supply conduit 160, can be used to transport gases from the gases source 110 to the humidifier 120. In some embodiments, an additional medical conduit, such as an interface conduit 170, can connect between the inspiratory conduit 140 and the user interface 180. In some embodiments, a connector 145 can connect between the inspiratory conduit 140 and the interface conduit 170. In some embodiments, exhaled gases can be transported via a medical conduit, such as an expiratory conduit 190. In a bubble CPAP respiratory therapy system 1 as shown in Figure 1, an end of the expiratory conduit 190 distal from the user is immersed in a water reservoir 195, the depth of the immersion in the reservoir 195 determining the pressure generated in the user's airways. In this system the integrated flow generator and humidifier generates and provides a gases flow at a set flow rate. Further the flow generator may be a blower. The system may comprise pressure relief valves for overpressure relief or the blower may be controlled to prevent an overpressure condition. For example the blower may be switched off if an over pressure is detected by a pressure sensor within the conduit or within the system housing.

Also add the exhaling of the patient causes bubbling within the reservoir, which can improve the CPAP therapy.

Figure 1 shows an example of how the respiratory therapy system 1 might be set up in an environment comprising an ambient environment and a controlled environment, where the inspiratory circuit passes through both environments. The controlled environment is shown inside the dashed line in Figure 1, and in this case comprises an incubator I which comprises part of the system 1. The system 1 shown is set up to provide bubble CPAP therapy, but could equally apply to other forms of therapy performed on a user placed inside a heated incubator I, such as nasal high flow therapy. The system 1 could equally be used in any other situation where the inspiratory circuit passes through an environment having varying environmental conditions along the length of the inspiratory circuit.

Initially, respiratory gases exit the humidifier 120 at point A at a set temperature and humidity (for example, a temperature of 40°C against a dewpoint of 37°C). The respiratory gases would then flow through the inspiratory conduit 140 to point C, located inside the incubator I (represented by the dashed box), at which point the temperature of the respiratory gases is measured. The power supplied to the heated inspiratory conduit 140 is regulated such that the temperature at point C matches the target temperature set by the system controller (for example 40°C). The controller could be in the main housing or enclosure of the device in which the flow generator is located, or may be at any other part of the system, or remote from the system and connected to the system 1 by wired or wireless communication. Similarly the system may comprise more than one controller.

Due to the presence of the incubator I in the example given, the section of the heated conduit 140 that is located outside of the incubator I (i.e. between points A and B) are exposed to a lower ambient temperature than the section of the heated conduit 140 that is located inside of the incubator I (i.e. between points B and C). This can result in the temperature dropping across the length of the heated conduit 140 between points A and B, before increasing to the target temperature across the length of the heated conduit 140 between points B and C. Depending on factors such as the temperature difference between the incubator I and the room air (i.e. a difference in a parameter between the controlled and ambient environments), it is possible that the temperature in the heated conduit 140 between points A and B may undesirably drop below the dewpoint at one or more locations.

### Respiratory Therapy System

This disclosure relates to modifying the Figure 1 type respiratory therapy system 1 to provide a respiratory therapy system 100 which includes another medical conduit 200 being an extension conduit with a first conduit connector 210 at a first end configured to connect to a connector 145 on the heated inspiratory conduit 140, and a second connector 220 at a second end configured to connect to a connector 185 on the user interface 180.

During use, substantially all of the entire length of the extension conduit 200 is located within the incubator I (that is, within the controlled environment), with substantially all of the entire length of the heated conduit 170 being located outside of the incubator I (that is, in the ambient or uncontrolled environment).

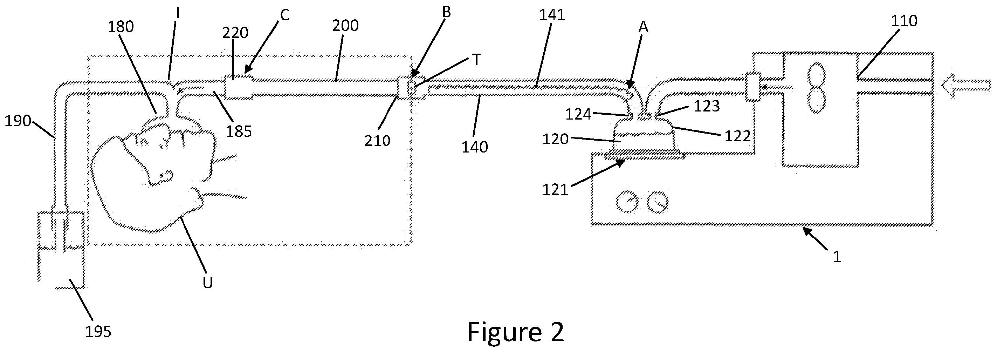

An example system 100 using the extension conduit 200 is shown in Figure 2. In this system 100, the heated inspiratory conduit 140 extends from the outlet 124 of the humidifier 120 (point A) to the boundary of the incubator I (point B) at which point it connects to the extension conduit 200. The extension conduit 200 extends from the boundary of the incubator I (point B) to the connector 185 of the user interface 180 (point C).

As described above, the power supplied to the heated inspiratory conduit 140 is controlled by a controller, such as the system controller, or any other local or remote controller, based on feedback from a temperature sensor T, located at point B, that is, located outside the incubator I, but as close to the incubator I as possible. As the entire length of the heated inspiratory conduit 140 is exposed to substantially the same ambient conditions, then the temperature of the respiratory gases throughout the entire length of the inspiratory conduit 140 should be above the dewpoint temperature, provided that the set target temperature for the conduit 140 is sufficiently high (as set by the controller in response to a signal indicative of temperature from the temperature sensor T).

After passing through the heated inspiratory conduit 140 the respiratory gases enter the extension conduit 200, located substantially within the incubator I (that is, within the controlled environment). The extension conduit 200 may be unheated. The extension conduit 200 may be breathable, as discussed in more detail below. The unheated, breathable conduit 200 provides a further length of conduit between the incubator and the interface conduit. The extension conduit 200 may be removably connectable between the inspiratory conduit 140 and the interface conduit 170. As the incubator I may be maintained close to or above the dewpoint temperature of the breathing gases in the inspiratory circuit, any temperature drop across the extension conduit 200 may be too small to cause condensation to occur. At the least the system 100 of Figure 2 should reduce condensate forming in the inspiratory circuit.

### Connectors

The extension conduit 200 comprises a first connector 210 at a first end configured to connect to a connector 145 on the heated inspiratory conduit 140. Additionally, the extension conduit 200 comprises a second connector 220 at a second end configured to connect to a connector 185 on the user interface 180.

Furthermore, the connector 145 on the heated inspiratory conduit 140 is configured to connect to the connector 185 on the user interface 180 such that the extension conduit 200 can be removed from the inspiratory circuit when not in use. As such, the first connector 210 may be substantially identical to the connector 185 on the user interface 180. The second connector 220 may be substantially identical to the connector 145 on the heated inspiratory conduit 140. Additionally, as the connector 145 on the heated inspiratory conduit 140 is complementary to the connector 185 on the user interface 180 in order to allow for the inspiratory circuit to be set up without the extension conduit 200, the first and second connectors 210, 220 on the extension conduit 200 are therefore compatible with each other, in that the connectors 210, 220 could be connected together.

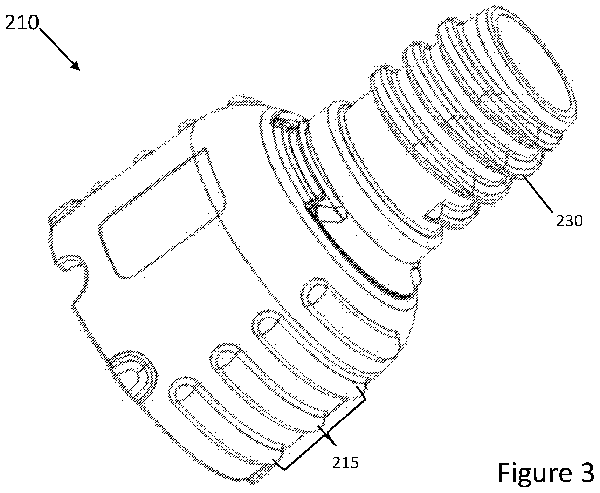

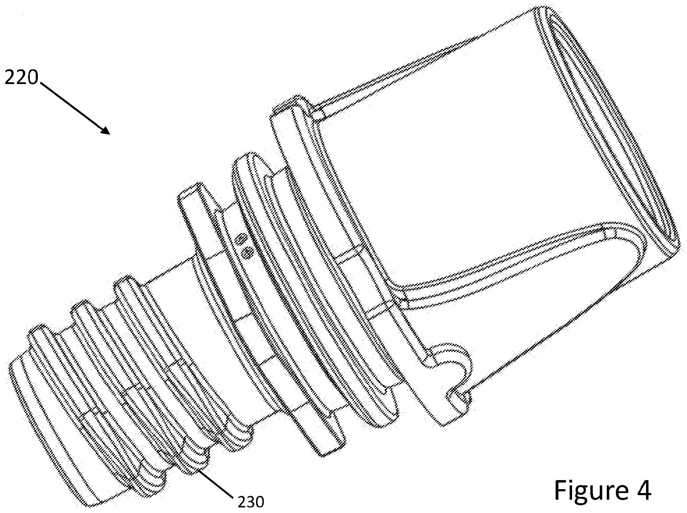

With reference to Figures 3 to 6, each connector 210, 220 connects to the flexible portion of a respective end of conduit 200 via a threaded connector portion 230 on each connector 210, 220. The thread (pitch and inner and outer diameters) on the threaded connector portion 230 corresponds to the internal thread (pitch and inner and outer diameters) of the flexible portion of conduit 200, and can be secured to the flexible portion using an adhesive for example. The threaded connector portion 230 of the connector 210, 220 may be constructed with a relatively slight taper (for example 2°) to allow for a more secure fit between the conduit 200 and the connectors 210, 220. The taper additionally causes a tighter fit at the base of the threaded connector portion 230, and as such the adhesive could be placed in this region. As a result, any expansion and/or swelling of the conduit 200 would be unlikely to cause the threaded connector portion 230 to detach from the flexible portion at the ends of the conduit 200.

As an alternative to the threaded connection, the flexible portion at the ends of the conduit 200 could be manufactured with a straight tubular portion at one or both ends. In this configuration the connectors 210, 220 could be constructed with a straight (non-threaded) taper to connect to the flexible portion at the ends of conduit 200. An adhesive may or may not be used in this scenario.

Other methods of attachment of the connectors 210, 220 to the conduit 200 may be used, including, for example, overmoulding part of the connectors 210, 220 to the conduit 200.

As can best be seen in Figure 3, the first connector 210 may have one or more protrusions or textured regions 215 to allow for greater grip when connecting and disconnecting the extension conduit 200 to/from the heated inspiratory conduit 140.

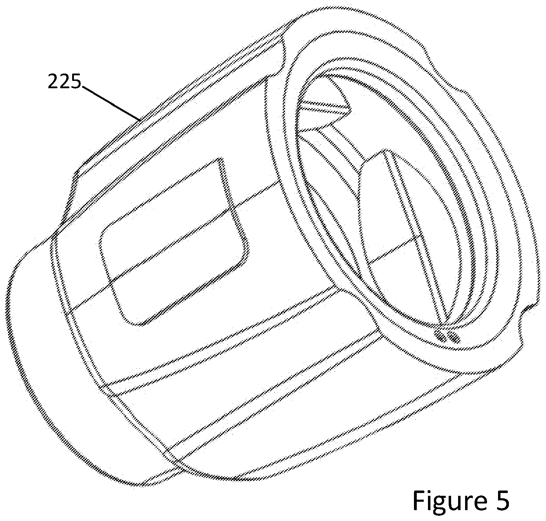

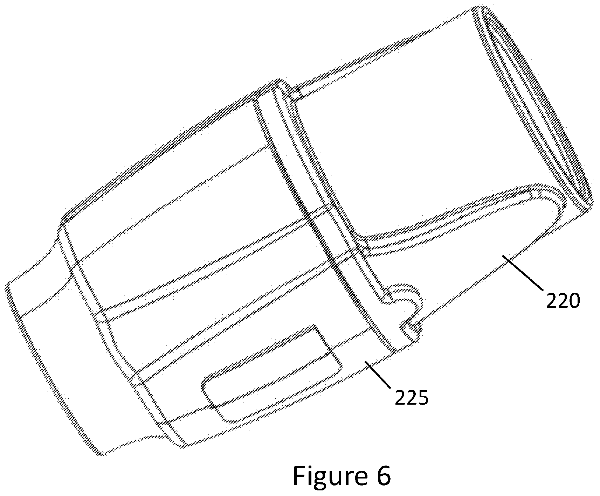

With reference to Figures 5 and 6, the second connector 220 can comprise an elastomeric outer sleeve 225. The elastomeric outer sleeve 225 allows for greater grip when connecting and disconnecting the extension conduit 200 to the user interface 180. The sleeve 225 could be constructed as a separate component, and then slid onto the connector 220 to form a composite connector assembly. Alternatively, the sleeve 225 could be overmoulded onto the connector 220. The outer sleeve 225 is configured to surround the outside of the flexible end portion of the extension conduit 200, where the conduit 200 is threaded onto the connector 220.

One example of such connectors 210, 220 are set out in our earlier patent application PCT publication WO2013/022356 (herein WO'356). In particular, the first and second connectors 210, 220 may comprise the locking fingers and locking tabs described in this publication.

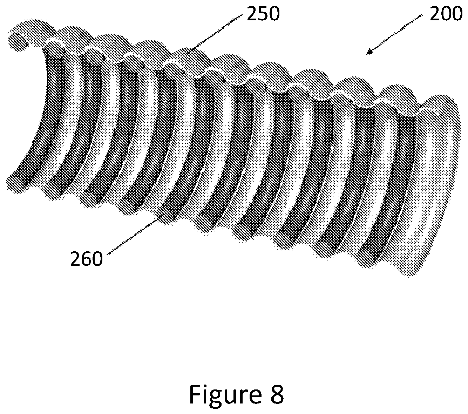

FIGS. 8A and 8B illustrate perspective views of the connector 210 or 220 from a source aperture 305 side and a terminal aperture 310 side, respectively, incorporating such locking fingers and locking tabs. In the illustrated embodiment, the connector 210 or 220 includes a substantially cylindrical conduit having two locking fingers 153 extending from the source aperture 305. The locking fingers 153 can be spaced apart The connectors 210, 220 may include a slot or opening to insert a temperature sensor within the connector 210, 220. The extension conduit 200 may also comprise a further sensor to measure the temperature at the end of the extension conduit 200. This second temperature sensor reading may be provided to the controller and the signals may be used to control the heater wire in the inspiratory conduit.

In some embodiments, each locking finger 153 includes a locking recess 154 formed on the outer surface of the locking finger 153. In one embodiment, the locking recesses 154 are configured to lock with the locking tabs of another connector, such as the gases conduit connector 145 or the user interface connector 185. In some embodiments, the locking fingers 153 include a flexible or semi-rigid material such that sufficient longitudinal force can cause the locking recesses 154 to pass over locking tabs 151 of the source conduit connector 125, thereby releasing the connecting adapter 140 from the source conduit connector 125. For example, pushing the connectors 210, 220 into gases conduit connector 145 or the user interface connector 185 (on assembly or connection) or pulling out the connectors 210, 220 (on disconnection) can cause the locking tabs of the gases conduit connector 145 or the user interface connector 185 to engage or disengage with the locking recesses 154 of the locking finger 153.

Also consider borrowing broad disclosure and drawings from WO2017/037660, e.g. figs 21-23.

Also include a disclosure of the connector in fig. 28 as a further alternative connector that could be used as the connector to couple to the inspiratory conduit connector.

### Conduit Structure

The length of the extension conduit 200 may be determined by two requirements. First, the extension conduit 200 needs to be long enough to reach from the periphery of an incubator enclosure of the incubator I to the user interface 180. In one example, the extension conduit 200 has a length at least equal to the distance between the periphery of the incubator enclosure, and the centre of the incubator. Secondly, a longer extension conduit 200 will lose a greater amount of heat along its length, as well as a greater amount of humidity if the conduit 200 is breathable (as is described below). As such, it is desirable to make the conduit 200 as short as possible, provided it is not too short to reach the user interface 180 or cause difficulty during the process of setting up the inspiratory circuit. In one configuration, the extension conduit length is between about 20cm and about 35cm. In a further configuration, the extension conduit length is about 25cm. In some configurations the extension conduit length may be between 10cm to 50cm, more preferably between 15cm to 40cm, even more preferably between 20cm and 35 cm.

The extension conduit 200 can be unheated. The extension conduit being unheated is advantageous because it reduces the chances of a patient (e.g. neonate) inside the incubator being burned. The external wall temperature of the extension tube does not rise to dangerous levels because the extension tube does not have a heater within it.

The extension conduit 200 also provides a further additional decoupling of the user interface 180 from the inspiratory conduit 140. The extension conduit 200 can move to reduce transfer of forces to the interface if the inspiratory conduit 140 is moved or moves in use. Further the extension conduit 200 can take movement of the interface conduit 140 and reduce transfer of forces to the inspiratory conduit 140 to prevent or reduce the chances of the inspiratory conduit 140 from being dislodged from an opening in the incubator I.

The extension conduit further helps to stabilise the inspiratory conduit and interface by decoupling the inspiratory conduit from the interface.

### Breathability

The extension conduit 200 can be made of a breathable material. An example of a breathable conduit is given in our earlier PCT application WO2016/048172 (herein WO'172).

"Breathable" as herein described refers to a part of the conduit that is highly permeable to moisture vapor such as water vapor, but is substantially impermeable to liquid moisture such as liquid water and substantially impermeable to the bulk flow of gases. Similarly, a "breathable material" generally refers to a material that is highly permeable to moisture vapor and substantially impermeable to liquid moisture and the bulk flow of gases. In certain embodiments, a breathable material has a moisture (water) vapor transmission rate greater than or equal to 650 g/m /day (or thereabout) when measured according to Procedure B of ASTM E96 (using the upright cup method at a temperature of 23 °C and a relative humidity of 50%).

In order to further reduce the chance of condensation occurring in the extension conduit 200, the extension conduit 200 could be made of a breathable material, or at least could comprise one or more portions of breathable material.

The breathable conduit 200 allows transfer of water vapour from within the conduit 200 to outside the conduit 200. This transfer of water vapour can occur due to a humidity gradient and/or temperature. The humidity is higher inside the conduit 200 and therefore some of the excess water vapour is transferred out of the extension conduit into the incubator environment, since it is "drier" than the conduit 200. This breathability reduces liquid condensate being formed. Additionally the breathable conduit 200 may also be made of a material that can absorb some amount of liquid water formed within the conduit 200. The absorption of liquid water (i.e. liquid condensate) reduces the build up of liquid water in the conduit 200.

In one example configuration the structure and/or material of the conduit 200 could be similar to that described in WO'172, an example of which can be seen with reference to Figure 8.

Figure 8 shows a medical conduit 200 configured to extend between the heated gases conduit 140 and the user interface 180 of the respiratory system 100. The medical conduit 200 can comprise an elongate film 250 and an elongate reinforcing member 260 that are extruded and spirally wound to form the medical conduit 200. In some embodiments, the elongate film 250 can be configured to be breathable such that condensate formed at, for example, the user interface 180, the connectors 145, 210 or the medical conduit 200 can be vaporised (e.g., by a heater such as a heater wire) and transferred through the elongate film 250 to the surrounding atmosphere if the condensate drains back to the medical conduit 200. The elongate reinforcing member 260 can provide rigidity and/or structural support to the elongate film 250. In some embodiments, the elongate reinforcing member 260 can comprise at least one wire, which can provide a heating and/or sensing component to the medical conduit 200. The conduit wall of the breathable material may be formed from a material that can absorb some amount of liquid water formed in the conduit 200. The liquid water may be absorbed into the wall of the conduit 200. The liquid may absorbed into the wall of the conduit 200 without heating. The unheated, breathable extension conduit 200 absorbs water into the wall of the conduit 200 to remove liquid water from the conduit 200.

The elongate film 250 can be in the range of about 50 and 200 µπι thick. In some embodiments, the elongate film 250 can be in the range of 50 and 75 µπι (or thereabout) thick. The thickness of the elongate film 250 can be important to reduce or eliminate the likelihood of the medical conduit 200 being damaged by the application of reasonable force. Reasonable force refers to a force that the medical conduit 200 is expected to encounter during normal use. Forces applied to the medical conduit 200 can be directed to the elongate film 250 and, thus, the elongate film 250 can determine the tensile strength of the medical conduit 200.

In certain embodiments, the elongate film 250 can have a tensile strength at 100% elongation greater than or equal to 650 lb/in (psi) (4.5 MPa) (or thereabout) and/or a tensile strength at 300% elongation greater than or equal to 1200 lb/in (8.3 MPa) (or thereabout). For example, the elongate film 250 can have a tensile strength at 100% elongation equal to 900 lb/in (6.2 MPa) and a tensile strength at 300% elongation equal to 1700 lb/in (1 1.7 MPa) (or thereabout). If the elongate film 250 is too thin it may be more vulnerable to damage, such as punctures or tearing, whereas if the elongate film 250 is too thick it may impair the breathability characteristics and may decrease the flexibility of the elongate film 250. Thus, the thickness of the elongate film 250 can be selected to consider the breathability, flexibility, and robustness of the elongate film 250 as desired for different applications. For example, the elongate film 250 can have a moisture (water) vapor transmission rate greater than or equal to 650 g/m /day (or thereabout) when measured according to Procedure B of ASTM E96 (using the upright cup method at a temperature of 23 °C and a relative humidity of 50%) and a tensile strength at 100% elongation greater than or equal to 650 lb/in (4.5 MPa) (or thereabout) and/or a tensile strength at 300% elongation greater than or equal to 1200 lb/in (8.3 MPa) (or thereabout). Insulating properties of the medical conduit 200 can also be important in reducing the amount of condensate within the medical conduit 200.

In some embodiments, the elongate film 250 can be made from a breathable thermoplastic material, such as a thermoplastic elastomer (or TPE as defined by ISO 18064:2003(E)), a thermoplastic polyurethane (or TPU as defined by ISO 18064:2003(E)), a thermoplastic polyester, or other material with elastomeric properties. The elongate reinforcing member 260 can be made from, for example, a TPU. The materials disclosed are not meant to be limiting but rather are examples of possible materials that can be used. The materials can be chosen such that a bond is formed between the elongate film 250 and the elongate reinforcing member 260. The materials can be chosen such that when the medical conduit 200 moves and/or contacts other surfaces, it remains quiet and unobtrusive. Different materials and/or material combinations can fall within the scope of this disclosure. The elongate film 250 can be wrapped around the outside of the elongate reinforcing member 260 such that the elongate reinforcing member 260 interacts with the lumen of the medical conduit 200 and the elongate film 250 forms the outer surface of the medical conduit 200. In some embodiments, wherein the elongate film 250 comprises a breathable material, this can allow more of the breathable surface of the elongate film 250 to be exposed to the environment such that a greater amount of moisture can be lost from the elongate film 250.

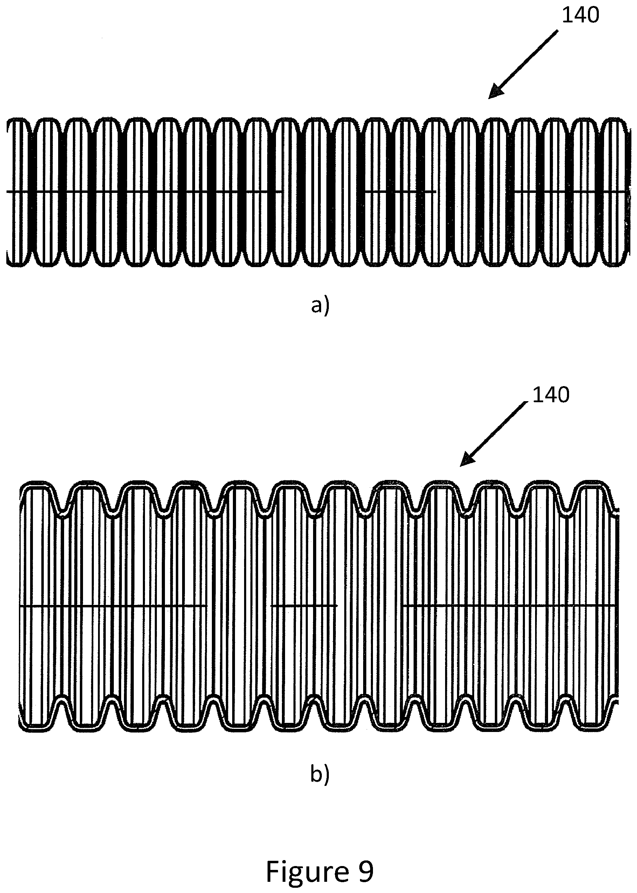

Additionally, or alternatively, the structure and/or material of the extension conduit 200 could be similar to that described in our earlier patent application PCT publication WO2011/077250 (herein WO'250), an example of which can be seen with reference to Figure 9.

Figures 9A and 9B show a breathable extension conduit 200 according to at least one embodiment. Figure 9A shows a side view of the conduit 200, while Figure 9B shows a longitudinal cross-section of the conduit 200. In both Figures, the horizontal axis is indicated as line L- L. The conduit wall, shown as wall 270 in Figure 9B is a breathable foamed material, as described above. Wall 270 can be between 100 and 1500 µ (or about 100 and 1500 µαι) thick for a breathing conduit of typical dimensions- between 12 and 20 mm (or about 12 and 20 mm) diameter for neonatal and adult applications respectively and 1 to 2 m (or about 1 to 2 m) in length. However, the wall 270 may be up to 3 mm (or about 3 mm) thick and still deliver good, or at least sufficient, breathability.

The conduit 200 is corrugated (that is, the conduit has a ridged or grooved surface). However, in some embodiments, the conduit has a smooth surface.

The conduit 200 could includes a plurality of reinforcing ribs that can be coextruded with the conduit 200. The form of the ribs may be determined by an extruder die head, and the size and the foaming level may be controlled by the temperature and pressure when it exits the die head.

The ribs can be formed from the same foamed polymer as the conduit 200. Alternatively, the ribs can be made from a different material than the conduit 200. This can be achieved by co-extrusion. The conduit 200 can be extruded with the ribs in place, and then corrugated to form a "dotted" structure. In certain embodiments, a conduit 200 includes between three and eight reinforcing ribs, such as between three and five reinforcing ribs.

An alternative configuration for a ribbed, breathable conduit 200 may provide raised ribs in the space between the ridges in the inside of the conduit 200. It will be appreciated that still other reinforcing processes may used to supplement the conduit 200 in order to improve its performance characteristics still further (such as compliance, pull strength, resistance to flow with bending and crush resistance). Those processes may or may not be integrated with the conduit forming process.

An possible construction of gases conduit 140, and/or extension conduit 200 is disclosed in WO2014077706.

This configuration of gases conduit 140/200 comprises pockets of air which provide insulation for the gases within the conduit which helps to reduce cooling of the gases in the conduit, thereby further reducing the chances of water vapour from the gases condensing within the conduit.

This configuration of conduit 140/200 comprises:
a. a bead
b. the bead may include heater wires.
c. the bead may include separate sensor wires.
d. the conduit comprises a pocket of air that acts as insulation.
e. the pocket of air and the bead are spirally wound to define the conduit with a hollow lumen therein to transport gases.

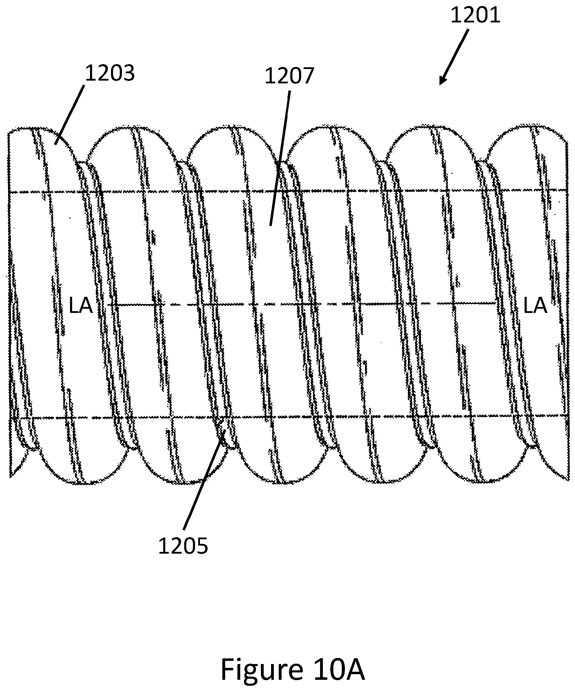

Figure 10A shows a side-plan view of a section of example conduit 1201 which can be used in conjunction with the respiratory humidification system 100 described with reference to Figure 2. The conduit 1201 can be used as part of the inspiratory circuit and can be configured, as described herein, to provide thermally beneficial properties that assist in the prevention of condensation of gases along the inspiratory circuit. The conduit 1201 includes a plurality of elongate members wrapped and joined to form a passageway, where the plurality of elongate members can include one or more of the heater wires described herein. Based at least in part on the heater wires being embedded in the walls of the conduit 1201, the use of the conduit 1201 as the inspiratory circuit can reduce condensation and rain out and maintain a more desirable or targeted temperature profile along the length of the inspiratory circuit. The conduit walls can provide a greater thermal mass which resists temperature changes and increases the insulating effects of the walls in relation to the ambient temperature outside the inspiratory circuit. As a result, the temperature along the length of the inspiratory circuit, including through any number of differing temperature environments, can be more accurately controlled and less power or energy can be expended in controlling the temperature of the gases delivered to the patient. In some embodiments, the conduit 1201 can be used as the expiratory circuit.

In general, the conduit 1201 comprises a first elongate member 1203 and a second elongate member 1205. Member is a broad term and is to be given its ordinary and customary meaning to a person of ordinary skill in the art (i.e., it is not to be limited to a special or customized meaning) and includes, without limitation, integral portions, integral components, and distinct components. Thus, although Figure 10A illustrates an embodiment made of two distinct components, it will be appreciated that in other embodiments, the first elongate member 1203 and second elongate member 1205 can also represent regions in a conduit formed from a single material. Thus, the first elongate member 1203 can represent a hollow portion of a conduit, while the second elongate member 1205 represents a structural supporting or reinforcement portion of the conduit which adds structural support to the hollow portion. The hollow portion and the structural supporting portion can have a spiral configuration, as described herein.

In this example, the first elongate member 1203 comprises a hollow body spirally wound to form, at least in part, an elongate conduit having a longitudinal axis LA- LA and a lumen 1207 extending along the longitudinal axis LA- LA. In at least one embodiment, the first elongate member 1203 is a conduit. Preferably, the first elongate member 1203 is flexible. Furthermore, the first elongate member 1203 is preferably transparent or, at least, semi-transparent or semi-opaque. A degree of optical transparency allows a caregiver or user to inspect the lumen 1207 for blockage or contaminants or to confirm the presence of moisture. A variety of plastics, including medical grade plastics, are suitable for the body of the first elongate member 1203. Examples of suitable materials include Polyolefin elastomers, Polyether block amides, Thermoplastic co-polyester elastomers, EPDM- Polypropylene mixtures, and Thermoplastic polyurethanes.

The hollow body structure of the first elongate member 1203 contributes to the insulating properties to the composite conduit 1201. An insulating conduit 1201 is desirable because, as explained herein, it prevents or reduces heat loss. This can allow the conduit 1201 to deliver gas from a heater-humidifier to a patient while substantially maintaining the gas's conditioned state with reduced or minimal energy consumption.

In at least one embodiment, the hollow portion of the first elongate member 1203 is filled with a gas. The gas can be air, which is desirable because of its low thermal conductivity (2.62x10*2 W/m- K at 300K) and very low cost. A gas that is more viscous than air may also advantageously be used, as higher viscosity reduces convective heat transfer. Thus, gases such as argon (17.72xl0"3 W/m-K at 300K), krypton (9.43xl0"3 W/m-K at 300K), and xenon (5.65xl0 3 W/ m-K at 300K) can increase insulating performance. Each of these gases is non-toxic, chemically inert, fire-inhibiting, and commercially available. The hollow portion of the first elongated member 1203 can be sealed at both ends of the conduit, causing the gas within to be substantially stagnant. Alternatively, the hollow portion can be a secondary pneumatic connection, such as a pressure sample line for conveying pressure feedback from the patient-end of the conduit to a controller. The first elongate member 1203 can be optionally perforated. For instance, the surface of the first elongate member 1203 can be perforated on an outward-facing surface, opposite the lumen 1207. In another embodiment, the hollow portion of the first elongate member 1203 is filled with a liquid. Examples of liquids can include water or other biocompatible liquids with a high thermal capacity. For instance, nanofluids can be used. An example nanofluid with suitable thermal capacity comprises water and nanoparticles of substances such as aluminium.

The second elongate member 1205 is also spirally wound and joined to the first elongate member 1203 between adjacent turns of the first elongate member 1203. The second elongate member 1205 forms at least a portion of the lumen 1207 of the elongate conduit. The second elongate member 1205 acts as structural support for the first elongate member 1203.

In at least one embodiment, the second elongate member 1205 is wider at the base (proximal the lumen 1207) and narrower at the top. For example, the second elongate member can be generally triangular in shape, generally T-shaped, or generally Y- shaped. However, any shape that meets the contours of the corresponding first elongate member 1203 is suitable.

Preferably, the second elongate member 1205 is flexible, to facilitate bending of the conduit. Desirably; the second elongate member 1205 is less flexible than the first elongate member 1203. This improves the ability of the second elongate member 1205 to structurally support the first elongate member 1203. For example, the modulus of the second elongate member 1205 is preferably 30 - 50MPa (or about 30 - 50 MPa). The modulus of the first elongate member 1203 is less than the modulus of the second elongate member 1205. The second elongate member 1205 can be solid or mostly solid. In addition, the second elongate member 1205 can encapsulate or house conductive material, such as filaments, and specifically heating filaments or sensors (not shown). Heating filaments can minimize the cold surfaces onto which condensate from moisture-laden air can form. Heating filaments can also be used to alter the temperature profile of gases in the lumen 1207 of composite conduit 1201. A variety of polymers and plastics, including medical grade plastics, are suitable for the body of the second elongate member 1205. Examples of suitable materials include Polyolefin elastomers, Polyether block amides, Thermoplastic co-polyester elastomers, EPDM-Polypropylene mixtures and Thermoplastic polyurethanes. In certain embodiments, the first elongate member 1203 and the second elongate member 1205 may be made from the same material. The second elongate member 1205 may also be made of a different colour material from the first elongate member 1203, and may be transparent, translucent or opaque. For example, in one embodiment the first elongate member 1203 may be made from a clear plastic, and the second elongate member 1205 may be made from an opaque blue (or other colour) plastic.

This spirally-wound structure comprising a flexible, hollow body and an integral support can provide crush resistance, while leaving the conduit wall flexible enough to permit short-radius bends without kinking, occluding or collapsing. Preferably, the conduit can be bent around a 25 mm diameter metal cylinder without kinking, occluding, or collapsing, as defined in the test for increase in flow resistance with bending according to ISO 5367:2000(E). This structure also can provide a smooth lumen 1207 surface (conduit bore), which helps keep the conduit free from deposits and improves gas flow. The hollow body has been found to improve the insulating properties of a conduit, while allowing the conduit to remain light weight.

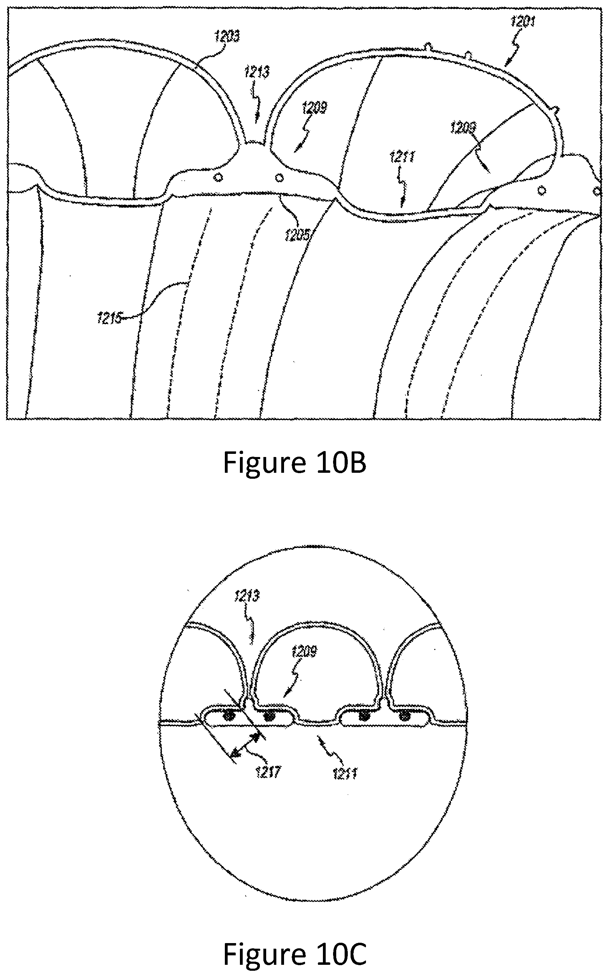

As explained above, the conduit 1201 can be used as an expiratory conduit and/or an inspiratory conduit in a breathing circuit, or a portion of a breathing circuit. Figure 10B shows a longitudinal cross-section of a top portion of the example composite conduit 1201 of Figure 10A. Figure 10B has the same orientation as Figure 10A. This example further illustrates the hollow-body shape of the first elongate member 1203. As seen in this example, the first elongate member 1203 forms in longitudinal cross-section a plurality of hollow bubbles. Portions 1209 of the first elongate member 1203 overlap adjacent wraps of the second elongate member 1205. A portion 1211 of the first elongate member 1203 forms the wall of the lumen (conduit bore).

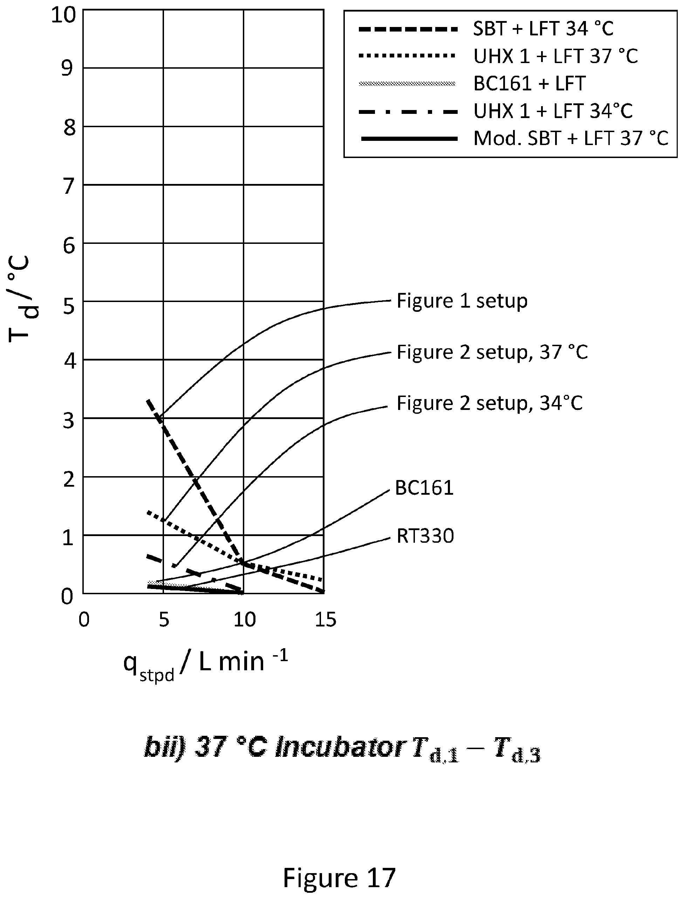

Having a gap 1213 between adjacent turns of the first elongate member 1203, that is, between adjacent bubbles, unexpectedly improved the overall insulating properties of the composite conduit 1201. Thus, in certain embodiments, adjacent bubbles are separated by a gap 1213. Furthermore, certain embodiments include the realization that providing a gap 1213 between adjacent bubbles increases the heat transfer resistivity (the R value) and, accordingly, decreases the heat transfer conductivity of the composite conduit 1201. This gap configuration was also found to improve the flexibility of the composite conduit 1201 by permitting shorter-radius bends. A T-shaped second elongate member 1205, as shown in FIG. 17B, can help maintain a gap 1213 between adjacent bubbles. Nevertheless, in certain embodiments, adjacent bubbles are touching. For example, adjacent bubbles can be bonded together.

One or more conductive materials can be disposed in the second elongate member 1205 for heating or sensing the gas flow. In this example, two heating filaments 1215 are encapsulated in the second elongate member 1205, one on either side of the vertical portion of the "T." The heating filaments 1215 comprise conductive material, such as alloys of Aluminium (Al) and/or Copper (Cu), or conductive polymer. Preferably, the material forming the second elongate member 1205 is selected to be non-reactive with the metal in the heating filaments 1215 when the heating filaments 1215 reach their operating temperature. The filaments 1215 may be spaced away from lumen 1207 so that the filaments are not exposed to the lumen 1207. At one end of the composite conduit, pairs of filaments can be formed into a connecting loop.

In at least one embodiment, a plurality of filaments are disposed in the second elongate member 1205. The filaments can be electrically connected together to share a common rail. For example, a first filament, such as a heating filament, can be disposed on a first side of the second elongate member 1205. A second filament, such as a sensing filament, can be disposed on a second side of the second elongate member 1205. A third filament, such as a ground filament, can be disposed between the first and second filaments. The first, second, and/or third filaments can be connected together at one end of the second elongate member 1205.

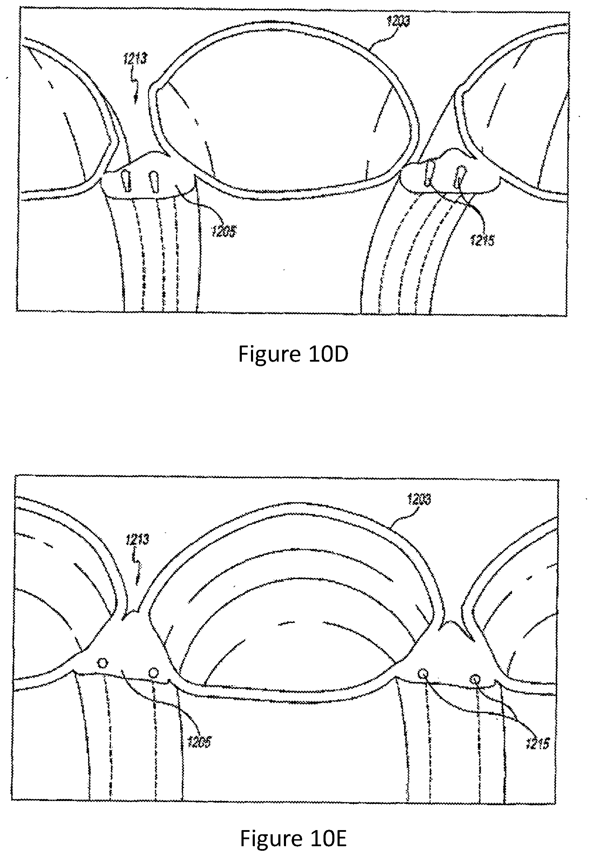

Figure 10C shows a longitudinal cross-section of the bubbles in Figure 10B. As shown, the portions 1209 of the first elongate member 1203 overlapping adjacent wraps of the second elongate member 1205 are characterised by a degree of bond region 1217. A larger bond region improves the conduits resistance to delamination at the interface of the first and second elongate members. Additionally or alternatively, the shape of the bead and/or the bubble can be adapted to increase the bond region 1217. For example, Figure 10D shows a relatively small bonding area on the left-hand side. In contrast, Figure 10E has a much larger bonding region than that shown in Figure 10B, because of the size and shape of the bead. It should be appreciated that although the configurations in Figure 10E may be preferred in certain embodiments, other configurations, including those of Figure 10D, and other variations, may be utilized in other embodiments as may be desired.

Figure 10D shows a longitudinal cross-section of a top portion of another composite conduit. Figure 10D has the same orientation as Figure 10B. This example further illustrates the hollow-body shape of the first elongate member 1203 and demonstrates how the first elongate member 1203 forms in longitudinal cross-section a plurality of hollow bubbles. In this example, the bubbles are completely separated from each other by a gap 1213. A generally triangular second elongate member 1205 supports the first elongate member 1203.

Figure 10E shows a longitudinal cross-section of a top portion of another composite conduit. Figure 10E has the same orientation as Figure 10B. In the example of Figure 10E, the heating filaments 1215 are spaced farther apart from each other than the filaments 1215 in Figure 10B. It was discovered that increasing the space between heating filaments can improve heating efficiency, and certain embodiments include this realization. Heating efficiency refers to the ratio of the amount of heat input to the conduit to the amount of energy output or recoverable from the conduit. Generally speaking, the greater the energy (or heat) that is dissipated from the conduit, the lower the heating efficiency. For improved heating performance, the heating filaments 1215 can be equally (or about equally) spaced along the bore of the conduit. Alternatively, the filaments 1215 can be positioned at extremities of the second elongate member 1205, which may provide simpler manufacturing.

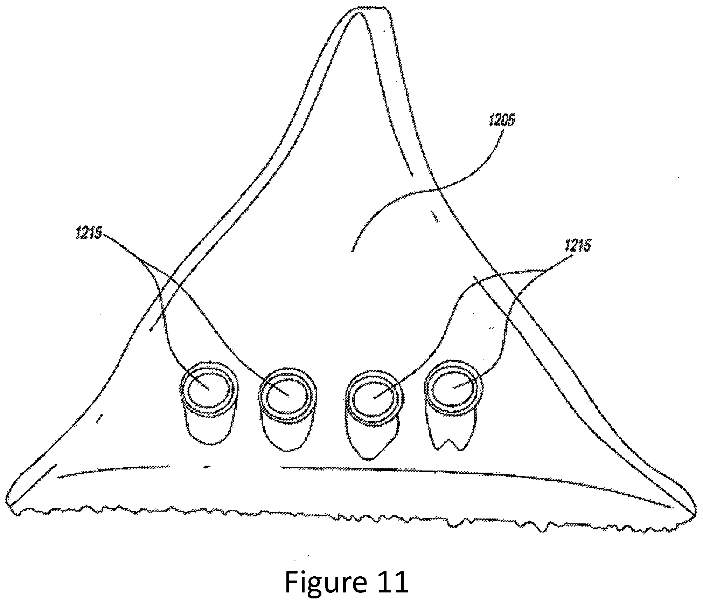

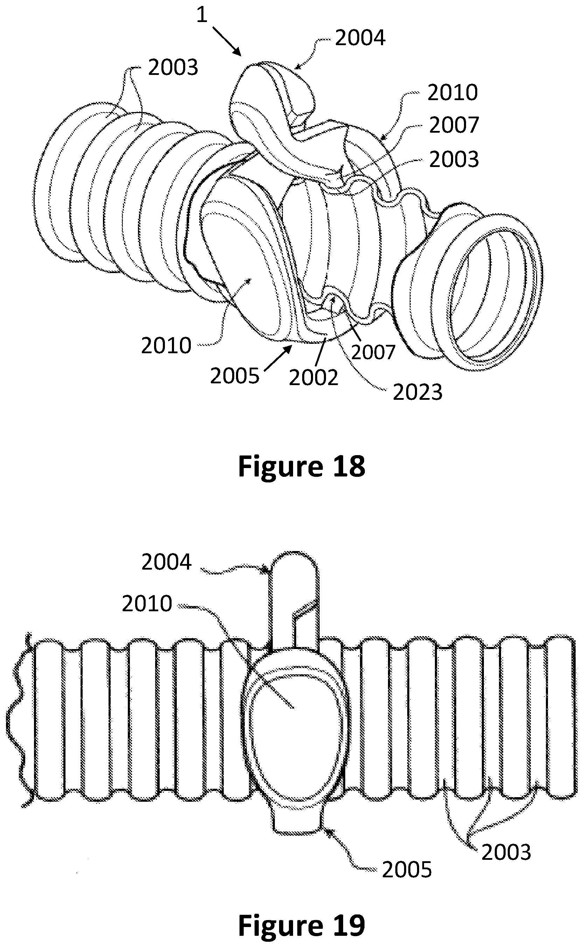

Reference is next made to Figure 11 which demonstrate example configurations for the second elongate member 1205. FIG. 18A shows a cross- section of a second elongate member 1205 having a shape similar to the T-shape shown in FIG. 17B. In this example embodiment, the second elongate member 1205 does not have heating filaments. Other shapes for the second elongate member 1205 may also be utilized, including variations of the T-shape as described below and triangular shapes.

Second elongate member 1205 could alternatively have a T-shape cross-section. In this example, heating filaments 1215 are embedded in cuts 1301 in the second elongate member 1205 on either side of the vertical portion of the "T." In some embodiments, the cuts 1301 can be formed in the second elongate member 1205 during extrusion. The cuts 1301 can alternatively be formed in the second elongate member 1205 after extrusion. For example, a cutting tool can form the cuts in the second elongate member 1205. Preferably, the cuts are formed by the heating filaments 1215 as they are pressed or pulled (mechanically fixed) into the second elongate member 1205 shortly after extrusion, while the second elongate member 1205 is relatively soft. Alternatively, one or more heating filaments can be mounted (e.g., adhered, bonded, or partially embedded) on the base of the elongate member, such that the filament(s) are exposed to the conduit lumen. In such embodiments, it can be desirable to contain the filament(s) in insulation to reduce the risk of fire when a flammable gas such as oxygen is passed through the conduit lumen.

Figure 11 shows yet another example second elongate member 1205 in cross-section. The second elongate member 1205 has a generally triangular shape. In this example four filaments 1215 are encapsulated in the second elongate member 1205, all of which are central in the bottom third of the second elongate member 1205 and disposed along a generally horizontal axis.

A possible configuration of connector 210/220 is as disclosed in WO2017/037660.

The connector 200, 40 comprises a body 50, the body comprising a first end 51 and a second end 52, the body 50 internally defining a lumen 53 for the passage of gas therethrough between each of the first and second ends. The first end 51, in use, being engaged or engageable with the terminal end of the gases conduit 140/200 or at least a component to be associated with the terminal end of the gases conduit 140/200. The connector 200 may be connected to the terminal end of the gases conduit 140/200 by a swivel-type connector connected to the body of the connector. The second end 52, in use, to be engaged or engageable with another connector (such as the connector of item 1 previously described herein). An internal surface of the body comprises one or more internal male connection features 53 extending therein configured for connection with a female end or female part of another connector receivable of said male connection features 53. The male internal connection features 53 comprising one or more (optionally a pair) of locking fingers 54. The connector 200, 40 comprising one or both of:
a. surrounding of the one or more internal male connection features 53 is an outer wall 55, an exterior surface 56 of the outer wall being tapered, tapered in a direction substantially longitudinally with the connector,
b. surrounding of the one or more internal male connection features 53 is an outer wall 55, the outer or at least an exterior surface 56 of the outer wall, comprising: one or more external alignment feature(s) 57 configured for aligning the connector 40 or another connector into an externally aligned connection therebetween, and/or one or more external visual aid(s) is/are configured for, in use, providing an externally visible guide for alignment of the connector or another connector into an aligned connection therebetween.

In some embodiments the first end of the connector 51 comprises a sleeve portion that flares outwardly. The outwardly flared portion allows for movement of a connected breathing conduit relative to the other connector, so that the gases conduit 140/200 may flex and bending without damage from the connector. Additionally, the outwardly flared sleeve portion also allows the user to better grip the connector for easier connection and disconnection with other connectors.

In some configurations, the one or more internal male connection features 53 is/are oriented so as to be radially aligned with the external alignment features 57 and/or the external visual aids. Each of these things in combination providing visual indicators, as well as physical aides to ensure a correct alignment of once connector with another connector.

The external alignment feature(s) 57 may be one or more cut-outs at a terminal end 58 or face of the outer wall 55 of the second end 52. The cut-outs can be configured to be received by a substantially reciprocally shaped portion on a connector to which said outer wall is to be placed into contact.

The various external alignment features 57 can be spaced, arrayed or arranged evenly or equidistantly from each other about the circumference a terminal end face of the outer wall of the second end.

The external alignment features 57 are configured to be co-located or co-locatable for keying with a reciprocally shaped projection of a sleeved portion of another connector when brought to bear into connection during a connection between a terminal face 58 of the second end 52 of the connector 200 and another connector.

The external alignment feature(s) 57 of the connector may be shaped or configured to prevent connection of the internal connection features 53 of the connector with another connector, when the external alignment feature(s) of the connector and an external alignment feature of another connector are in an unaligned orientation.

Additionally or alternatively the external alignment feature(s) 57 of the connector may be shaped or configured to allow connection of the internal connection features 53 of the connector with another connector (for example connector 1), when the external alignment feature(s) 57 of the connector and an external alignment feature of another connector are in an aligned orientation.

The locking fingers 54 may comprise a recess 61 on an outer surface of each finger 54. Such a recess 61 provided for receiving or engaging with an internal connection feature, such as a raised protrusion or tab such as a locking tab 14 of a connector such as that indicated as item 1. The recess 61 can be shaped for receipt of the internal connection feature of another connector.

A tip 62 of the locking fingers may be of an at least partially chamfered configuration, preferentially to assist in the locking fingers 54 being able to be received into engagement by a connection feature.

Various shapes of the alignment feature 57 as a cut out may be provided, for example: semi-circular, triangular, rectangular or other recti-linear or geometric shapes, elliptical, wedge shaped.

In various configurations, the outer wall 55 operates as a sleeve, for being brought into a sleeved connection with another connector (whether as a male or female connection). In one embodiment, the outer wall is configured for use as a 22mm male taper connector to another connector comprising a female connection facility, and in another the outer wall is a sleeve, configured for use as a 22 female taper connector to another connector comprising a male connection facility.

The connectors or at least their body may be formed of any medically suitable materials, however particularly preferred is Polycarbonate (PC),

Polyethylene (PE), Acryionitrile Butadiene Styrene (ABS) or polypropylene (PP).

The internal surface of the second end 52 of the body may further comprise a protrusion for an engagement (e.g. an interference fit) with a commensurately shaped portion of another connector to be received by or within the internal surface bounded by the outer wall 55. For example, such a protrusion can extend as a shoulder 63 radially outwardly from the one or more locking fingers 54, or from a base 64 extending as a floor from the inner surface of the outer wall 55. The shoulder may also optionally also extending longitudinally in a direction toward an open end of the second end of the connector. Or, alternatively or in addition, an inner surface of the outer wall may comprise of a radially inwardly extending protrusion 60a. A space 59 is defined between a radially outward surface of the shoulder 63 and an inner surface of the outer wall or the inwardly extending protrusion 60a, such a space being sized and shaped so as to be receivable of a terminal end of another connector, such as a lip or flange part of another connector. The terminal end of the another connector can be received in the space as an interference fit between the outward surface of the shoulder and the inner surface of the outer wall or the protrusion 60a. For example see Figure 32 shows an assembly of a lip or flange (or a shoulder) 91 of another connector 90 which can be brought into such an interference fit in the space 59 with a connector such as that itemised as connector 200. In this manner, the lip or flange 91 can be retained or held in place by the interference fit, and can also provide for a suitable pneumatic seal between each of these parts.

Alternatively or additionally, substantially adjacent to, or at least in part abutting the shoulder 63 and the inner surface of the outer wall, is a base 64, the base extending as a floor between an outward periphery of the shoulder and the inner surface of the wall. The base 64 may define a sealing surface upon which a terminal end or a face of a terminal end of another connector may become engaged therewith, optionally forming a pneumatic connection.

The connector 200 is configured to provide for a plurality of separate sealing surfaces upon which seals may be made, whether as a seal between component parts along the entirety of a surface or a point or particular location along that surface. As such, the connector 200 provides for six separate and different sealing surface options. Each of which are described below.

Constructing the extension conduit 200 from a breathable material allows the conduit 200 to release excess humidity to the surrounding environment. This may relatively slightly reduce the dewpoint of the respiratory gases in the inspiratory circuit, such that a corresponding relatively slight drop in temperature would not result in condensation forming.

The extension conduit 200 may therefore be provided as an incubator gas delivery conduit, configured to be located in an incubator I, as an extension of the inspiratory part of the breathing circuit that extends from a humidifier of the respiratory therapy system, to the user interface worn by the user.

The incubator gas delivery conduit 200 is configured to extend from the periphery of the incubator I, into the interior chamber of the incubator I. The incubator gas delivery conduit 200 (i.e. medical extension conduit) is configured to extend from the connection with the heated inspiratory conduit.

The medical extension conduit 200 may therefore be provided as an environmental gas delivery conduit of the inspiratory part of the breathing circuit, configured to be located in a location or region having different environmental conditions from a heated gases conduit of the breathing circuit, for example a location or region subject to a different temperature.

The incubator I may be considered to be a device for providing controlled environmental conditions, that differ from the ambient conditions of the environment in which the device is located. The device may be considered to comprise part of the respiratory therapy system 100, or may be considered to be a standalone device configured to be used with the respiratory therapy system 100.

The system 100 or device may comprise the extension conduit 200, or the extension conduit may be supplied as part of an inspiratory circuit or full breathing circuit kit for use with the system 100 or device. For example the extension conduit 200 may be supplied as part of a kit comprising any one or more of the user interface, heated gases conduit, a humidifier water or liquid chamber of the humidifier, one or more conduit connectors, and/or a connector elbow configured to connect the conduit 200 to the user interface.

Advantages of the extension conduit 200 include, for example, reducing liquid condensate by allowing transfer of excess water vapour as the temperature of the gases reduces or drops below dew point. Further the extension conduit 200 can absorb some amount of liquid water into the wall of the conduit to reduce the chances of further liquid condensate forming in the conduit and reducing any obstructions to the gases delivery. Further the extension conduit 200 can provide decoupling of the inspiratory conduit from the interface.

The removable extension conduit may also advantageous because it can be removably connected between an interface conduit and the inspiratory conduit. The extension conduit comprises a first connector on one end and a second connector on the opposing end that allows connection with complementary connectors of the inspiratory conduit and the interface conduit. The removable extension conduit (i.e. extension conduit) allows for a modular breathing circuit, wherein the extension conduit can be optionally connected between the interface conduit and the inspiratory conduit if needed. The need can arise when using an incubator with the inspiratory conduit. This is advantageous because the extension conduit can be optionally included in the circuit where there are changes in the environment in which the incubator is used, and to reduce the chances of liquid condensate forming in the circuit.

The extension conduit also provides a connecting conduit to couple the inspiratory conduit to the interface conduit. This is advantageous because the inspiratory conduit and interface conduit do not need to be modified. Further this is advantageous because the interface conduit is not stretched and the increased length of the circuit (due to the extension conduit) allows the neonate patient to move around while decoupling the interface from the inspiratory conduit and absorbs movement of the infant. Further the modular nature of the circuit and increased length of the circuit allows the infant to be placed anywhere within the incubator while still connecting the interface with the inspiratory conduit.

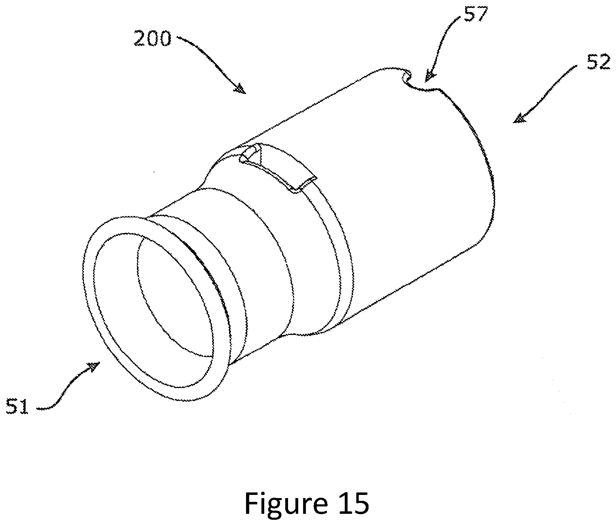

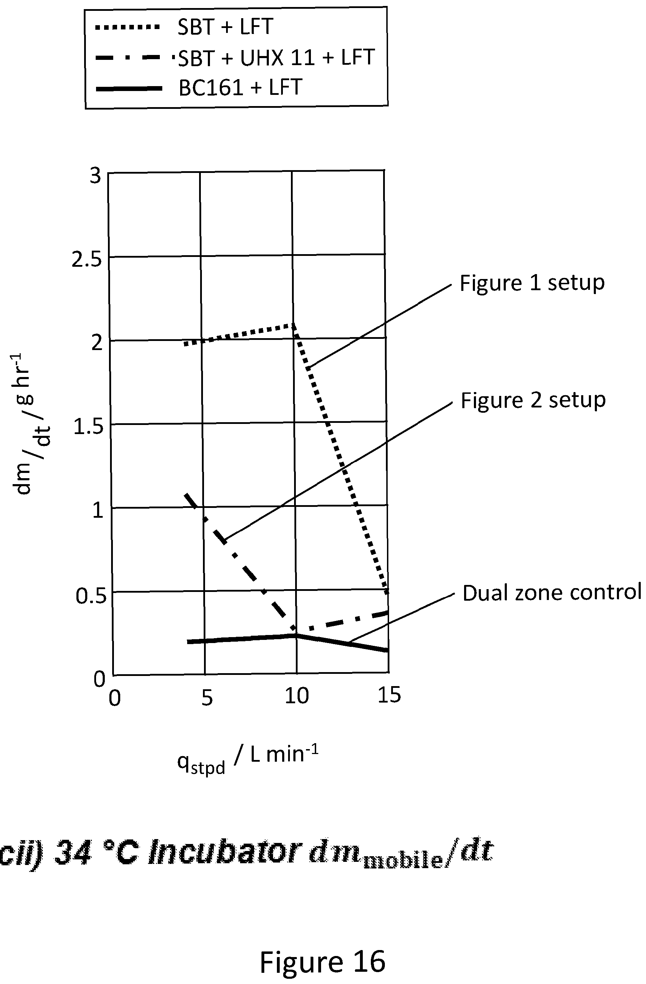

Referring now to Figures 15 and 16, these show graphs of test data taken from an extension conduit 200 in accordance with this disclosure.

The graph of Figure 15 shows the humidity drop against flow rate for various setups. At high flow rates the humidity drop is relatively low as the gas moves through the conduit relatively quickly. At low flow rates, the largest humidity drop is seen in the setup described in Figure 1 of the patent, as shown by the red line. This humidity drop is caused by the temperature sensor being inside the incubator I, thereby resulting in condensation as described above. Following this is the humidity drop in the unheated extension conduit 200, the blue line, which is largely caused by the breathability of the inspiratory circuit. The lowest humidity drop is seen in standard bubble CPAP circuit, which uses dual zone temperature control when in an incubator I, the green line.

The graph of Figure 16 shows the temperature drop against flow rate for various setups. Distinction here is made as well between the target temperature for each setup as well (note that the target temperature is not the actual temperature that the conduit is controlled to but instead the target dewpoint, the actual temperature that the system is controlled to is higher than this to provide a buffer zone with the dewpoint that reduces the risk of condensate).

The largest temperature drop is for the setup described in Figure 1 of the patent with a target temperature of 34°C, the red line. Because of the lower target temperature combined with the temperature sensor being in the incubator, a low amount of power is supplied to the heated breathing conduit 140. This low amount of power results in a large temperature drop, which in turn results in the condensation evident in Figure 15.

Following this is the temperature drop for the unheated breathable extension conduit 200 with a target temperature of 37°C can be seen with reference to the blue line. Whilst there is a noticeable drop in temperature, this should not be enough to cause condensate as the dewpoint will have also decreased due to the breathability of the conduit 200.

Following this is the temperature drop for the unheated breathable extension with a target temperature of 34°C as per the pink line. The situation here is similar to the previously described setup, however with a lower temperature drop caused by a lower target temperature resulting in a lower temperature difference between the gases flow and the incubator.

Only a negligible amount of temperature drop is seen in the RT330 circuit (the blacked line) and BC161 circuit (the green line), both of which use dual zone temperature control when in an incubator.

As explained above, it can be desirable to prevent or resist movement of the patient inside the incubator moving the connector between the unheated medical conduit inside the incubator, and the heated inspiratory conduit outside the incubator.

Referring to Figures 18 and 19, a component 2001 is provided for use with medical conduit 200 or heated inspiratory conduit 140. The component 2001 comprises a body 2002 engageable with one or more external surface recesses 2003 (or surface relief portions or sections) of a conduit 200/140 (e.g., such as those of a corrugated conduit or conduit with a helically recessed surface region).

The component 2001 includes a pair of jaws 2004 that extend from the body 2002. The jaws 2004 can be used for attaching to or for gripping of an item (not shown). In use, in a first orientation 2005 of the body 2002 relative to the conduit recesses 2003, the component 2001 is movable along a length of the conduit 200/140. While in a second orientation 6 of the body 2 relative to the conduit recesses 3, the component 1 is resistive to movement along a length of the conduit 200/140.

The body 2002 substantially surrounds the perimeter, or circumference, of the conduit 200/140, such that a portion or portions (such as projection 2007 or 2020, 2022 or 2030) of the body 2002 are engageable with the surface recesses 2003 or with surface relief portions or sections. Accordingly, the component 2001 is enabled to be moved along the conduit 200/140 when in a first body orientation, yet the component 2001 being resistive to being moved along the conduit 00/140 when in a second body orientation relative to the conduit's recesses 2003 or surface relief features.

As a part of substantially surrounding the conduit 200/140, an internal surface (or surfaces or portions of the internal surface) of the body 2002 is or are engageable with the one or more external surface recesses 2003 (or relief features) of the conduit 200/140. In this manner, the body 2002 may be substantially annular about the exterior surface of the conduit 200/140 being engaged.

Turning to the jaws 4 of the component 1, in one preferred embodiment the jaws 2004 are configured to be opposing upon one another, or co-acting on each other, when in their closed position 2008. Such jaws 2004, or other variations of these, may be configured such that they are hingedly biased toward each other in reaching a substantially closed position 2008. Further, the jaws 2004 can be openable from a substantially closed position 2008 to enable the jaws 2004 to receive and then close upon an item for gripping of that item. The jaws 2004 can be openable by deflecting the jaws 2004 away from each other. Manually actuating the jaws 2004 to create such a deflection or opening is additionally facilitated by providing finger grips or tabs for the user of the component 2001. The jaws 2004 can clamp onto a sheet/mattress of the incubator for example, or another fixed item inside the incubator.

The above disclosure therefore provides a clip component configured to engage the conduit 200/140, and when so engaged to resist relative movement between the conduit 200/140 and the clip component 2001 generally along the direction of the conduit longitudinal axis. When so engaged, the clip component 2001 can, for example secure the breathable medical conduit 200 to a sheet/mattress of the infant warmer/incubator,

The provision of respiratory therapy for a patient in an incubator presents a number of challenges and conflicting requirements. It is desirable to be able to heat the respiratory gases provided to the patient, but it is undesirable to further heat the incubator. Hence it is undesirable to locate the heated inspiratory conduit inside the incubator. But not doing so can have the effect that the respiratory gases cool as they enter and pass through the incubator, potentially causing condensate to form. Such condensate, in the breathable gases flow to the patient, is undesirable. The provision of a medical conduit as described above, which may be unheated and/or breathable, assists in dealing with one or more of these problems. Providing such a medical conduit can also assist in preventing or resisting the patient interface from being disconnected from the heated inspiratory conduit, for example, during movement of the patient when inside the incubator.

Unless the context clearly requires otherwise, throughout the description, the words "comprise", "comprising", and the like, are to be construed in an inclusive sense as opposed to an exclusive or exhaustive sense, that is to say, in the sense of "including, but not limited to".

Where reference is used herein to directional terms such as 'up', 'down', 'forward', 'rearward', 'horizontal', 'vertical' etc., those terms refer to when the apparatus is in a typical in-use position, and are used to show and/or describe relative directions or orientations.

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to".

The terms "approximately," "about," and "substantially" as used herein represent an amount close to the stated amount that still performs a desired function or achieves a desired result. For example, in some embodiments, as the context may permit, the terms "approximately", "about", and "substantially" may refer to an amount that is within less than or equal to 10% of, within less than or equal to 5% of, and within less than or equal to 1% of the stated amount.

Reference to any prior art in this specification is not, and should not be taken as, an acknowledgement or any form of suggestion that that prior art forms part of the common general knowledge in the field of endeavour in any country in the world.

The disclosed apparatus and systems may also be said broadly to consist in the parts, elements and features referred to or indicated in the specification of the application, individually or collectively, in any or all combinations of two or more of said parts, elements or features.

Where, in the foregoing description reference has been made to integers or components having known equivalents thereof, those integers are herein incorporated as if individually set forth.

Depending on the embodiment, certain acts, events, or functions of any of the algorithms, methods, or processes described herein can be performed in a different sequence, can be added, merged, or left out altogether (for example, not all described acts or events are necessary for the practice of the algorithms). Moreover, in certain embodiments, acts or events can be performed concurrently, for example, through multi-threaded processing, interrupt processing, or multiple processors or processor cores or on other parallel architectures, rather than sequentially.

It should be noted that various changes and modifications to the presently preferred embodiments described herein will be apparent to those skilled in the art. Such changes and modifications may be made without departing from the spirit and scope of the disclosed apparatus and systems and without diminishing its attendant advantages. For instance, various components may be repositioned as desired. It is therefore intended that such changes and modifications be included within the scope of the disclosed apparatus and systems. Moreover, not all of the features, aspects and advantages are necessarily required to practice the disclosed apparatus and systems. Accordingly, the scope of the disclosed apparatus and systems is intended to be defined only by the claims that follow.

The present disclosure extends to the following numbered clauses:
1. A medical conduit configured to deliver breathable gases in a respiratory therapy system; the medical conduit comprising:
   i. a first conduit end connector configured to be connected to a user interface;
   ii. a second conduit end connector configured to be connected to a heated inspiratory conduit;
   iii. the medical conduit further comprising at least one portion intermediate the first and second conduit end connectors made from a breathable material;
   iv. the medical conduit being configured to connect the user interface to the heated inspiratory conduit;
   the medical conduit being configured, when connected to the user interface and the heated inspiratory conduit, to be located in an incubator.
2. A medical conduit configured to deliver breathable gases in a respiratory therapy system; the medical conduit comprising:
   i. a first conduit end connector configured to be connected to a user interface;
   ii. a second conduit end connector configured to be connected to a heated inspiratory conduit;
   iii. the medical conduit further comprising at least one portion intermediate the first and second conduit end connectors made from a breathable material;
   iv. the medical conduit being configured to connect the user interface to the heated inspiratory conduit;
   v. the medical conduit being configured, when connected to the user interface and the heated inspiratory conduit, to be located in an incubator; wherein
   vi. the medical conduit is unheated.
3. An incubator breathing gas delivery conduit configured to deliver breathable gases in a respiratory therapy system including an incubator; the medical conduit comprising:
   i. a first conduit end connector configured to be connected to a user interface inside the incubator;
   ii. a second conduit end connector configured to be connected to a heated inspiratory conduit outside the incubator;
   iii. the incubator breathing gas delivery conduit further comprising at least one portion intermediate the first and second conduit end connectors made from a breathable material.
4. The conduit of clause 1 or 3 wherein the conduit is unheated, that is, the medical conduit does not comprise any heater to heat gases flowing through the medical conduit.
5. The conduit of any one of the preceding clauses comprising one or more thermally insulating portions, for example formed by one or more air pockets or other insulating material.
6. The conduit of clause 1 to 5 comprising an elongate film spirally wrapped with an elongate reinforcing member to form a lumen, the elongate film bonding with the elongate reinforcing member.
7. The conduit of clause 6 wherein the at least one portion of the medical conduit which is made from breathable material comprises at least one portion of the elongate film.
8. The conduit of any one of the preceding clauses wherein the conduit is compressible, that is, the length of the conduit can be reduced.
9. The conduit of any one of the preceding clauses wherein the conduit is extensible, that is, the length of the conduit can be increased.
10. The conduit of any one of the preceding clauses wherein the conduit is breathable in that at least part of the conduit is highly permeable to moisture vapor such as water vapor, but is substantially impermeable to liquid moisture such as liquid water and substantially impermeable to the bulk flow of gases.
11. The conduit of any one of the preceding clauses wherein the length of the conduit is between about 20cm and 35cm, and in some configurations is about 25cm.
12. The conduit of any one of clauses 1 to 10 wherein the length of the conduit is between 10cm and 50cm, in some configurations between 15cm and 40cm, and in some configurations between 20cm and 35 cm.
13. The conduit of any one of the preceding clauses wherein the length of the conduit is less than the length of the heated inspiratory conduit with which the conduit is used.
14. A respiratory therapy kit for use in a respiratory therapy system, the kit comprising:
   i. a conduit according to any one of clauses 1 to 13;
   ii. a user interface configured to be secured to a user's head to deliver breathable gases to the user; and
   iii. a heated inspiratory conduit configured to receive humidified breathable gases from a humidifier of the respiratory therapy system.
15. The respiratory therapy kit of clause 14 wherein the user interface comprises an interface conduit.
16. The respiratory therapy kit of clause 14 or 15 wherein the conduit comprises:
   i. a first conduit end connector configured to be connected to a connector of the user interface, and a second conduit end connector configured to be connected to an end connector of the heated inspiratory conduit; wherein
   ii. the first conduit end connector is also configured to be connectable to the second conduit end connector.
17. A respiratory therapy kit of clause 14 or 15 wherein the conduit comprises:
   i. a first conduit end connector configured to be connected to a connector of the user interface, and a second conduit end connector configured to be connected to an end connector of the heated inspiratory conduit; wherein
   ii. the end connector of the heated inspiratory conduit is configured to be connected directly to the connector of the user interface, without using the medical conduit.
18. A respiratory therapy kit for use in a respiratory therapy system, the kit comprising:
   i. the conduit of any one of clauses 1 to 13; and
   ii. a heated inspiratory conduit configured to receive humidified breathable gases from a humidifier of the respiratory therapy system;
   iii. the medical conduit comprising:
      i. a first conduit end connector configured to be connected to a connector of a user interface of the respiratory therapy system, and a second conduit end connector configured to be connected to an end connector of the heated inspiratory conduit; wherein the first conduit end connector is also configured to be connectable to the second conduit end connector.
19. A respiratory therapy kit for use in a respiratory therapy system, the kit comprising:
   i. the conduit of any one of clauses 1 to 13; and
   ii. a heated inspiratory conduit configured to receive humidified breathable gases from a humidifier of the respiratory therapy system;
   iii. the medical conduit comprising:
      a first conduit end connector configured to be connected to a connector of a user interface of the respiratory therapy system, and a second conduit end connector configured to be connected to an end connector of the heated inspiratory conduit; wherein
   the end connector of the heated inspiratory conduit is configured to be connected directly to a connector of the user interface, without using the medical conduit.
20. The respiratory therapy kit of any one of clauses 14 to 19 wherein the heated inspiratory conduit comprises a temperature sensor, configured to measure the temperature of breathable gases in the heated inspiratory conduit.
21. The respiratory therapy kit of any one of clauses 14 to 20 wherein the connector on the user interface is substantially identical to the second conduit end connector of the conduit.
22. An incubator comprising the conduit of any one of clauses 1 to 13; and/or a respiratory therapy kit of any one of clauses 14 to 21.
23. The incubator of clause 22 wherein the heated inspiratory conduit comprises a temperature sensor, configured to measure the temperature of breathable gases in the heated inspiratory conduit.
24. The incubator of clause 23 comprising a controller, wherein the controller uses an output of the temperature sensor to control the heat delivered to the breathable gases by the heated inspiratory conduit, the controller controlling the heat delivered based on the dewpoint of the breathable gases.
25. The incubator of clause 24 wherein the temperature sensor is positioned outside of the incubator.
26. The incubator of any one of clauses 22 to 25 wherein the conduit and the heated inspiratory conduit care configured such that the conduit is inside the incubator, and the heated inspiratory conduit is outside of the incubator.
27. The incubator of clause 26 wherein the second conduit end connector of the conduit is positioned outside the incubator, when connected to the heated gases conduit.
28. The incubator of clause 26 or 27 wherein the second conduit end connector of the conduit is positioned inside the incubator, when connected to the heated gases conduit.
29. The incubator of any one of clauses 22 to 28, wherein the incubator has an incubation enclosure in which the user is contained in use, and the medical conduit has a length, the length being sufficient to extend from a periphery of the incubation enclosure to the centre of the incubation enclosure.
30. A respiratory therapy system comprising:
   i. a flow generator;
   ii. a humidifier;
   iii. a heated inspiratory conduit;
   iv. a user interface coupled to the gases conduit to deliver a gases flow to a user;
   v. a sensor configured to determine pressure or flow of the gases flow;
   vi. a controller configured to control the flow generator to generate the gases flow;
   vii. the system further comprising the conduit of any one of clauses 1 to 13; and/or a respiratory therapy kit of any one of clauses 14 to 21.
31. The respiratory therapy system of clause 30 wherein the heated inspiratory conduit comprises a temperature sensor, configured to measure the temperature of breathable gases in the heated inspiratory conduit.
32. The respiratory therapy system of clause 31 wherein the controller uses an output of the temperature sensor to control the heat delivered to the breathable gases by the heated inspiratory conduit, the controller controlling the heat delivered based on the dewpoint of the breathable gases.
33. The respiratory therapy system of any one of clauses 30 to 32 configured for use in an environment comprising both an ambient environment, subject to ambient environment conditions, and a controlled environment subject to at least one controlled parameter, wherein the gases conduit medical conduit is configured to be located in the ambient environment, and the medical conduit is configured, when connected to the gases conduit, to be located in the controlled environment.
34. The respiratory therapy system of clause 33 wherein the controlled parameter is selected from any of:
   i. temperature;
   ii. humidity;
   iii. pressure;
   iv. flow.
35. The respiratory therapy system of any one of clauses 30 to 34 wherein the breathable material is configured to manage condensation within the conduit, the breathable material allowing transmission of water vapor out of the conduit while reducing or preventing transmission of liquid water out of the conduit.
36. The respiratory therapy system of clause 35 wherein the breathable material is configured to absorb liquid water into the conduit.
37. The respiratory therapy system of clause 35 or 36 wherein the breathable material reduces condensate in the conduit.

## Claims

1. A respiratory therapy assembly for use in a respiratory therapy system, comprising:
an unheated medical extension conduit configured for placement within an incubator, the medical extension conduit comprising a first end connector and a second end connector;
a user interface connectable to the second end connector, the user interface configured to be secured to a user's head to deliver breathable gases to the user;
a heated inspiratory conduit configured to receive humidified breathable gases from a humidifier of the respiratory therapy system, the heated inspiratory conduit configured to be connected to the first end connector;
a connector arrangement configured to couple the heated inspiratory conduit to the first end connector;
wherein the connector arrangement is configured to be positioned at or adjacent a boundary of the incubator between an interior incubator environment and an exterior environment;
wherein the connector arrangement is configured to releasably connect the heated inspiratory conduit to the medical extension conduit; and
a clip member, configured to engage the heated inspiratory conduit or the medical extension conduit, or both, to the incubator, to resist displacement of the connector arrangement away from the boundary of the incubator during movement of the user.

2. The respiratory therapy assembly of claim 1, wherein substantially the entire length of the heated inspiratory conduit is positioned outside the incubator and substantially the entire length of the medical extension conduit is positioned inside the incubator.

3. The respiratory therapy assembly of claim 1 or claim 2, wherein the medical extension conduit comprises a breathable wall configured to transmit water vapour while substantially preventing bulk gas flow through the wall.

4. The respiratory therapy assembly of any preceding claim, wherein the medical extension conduit comprises no heater configured to heat the gases flowing through the medical extension conduit.

5. The respiratory therapy assembly of any preceding claim, wherein the heated inspiratory conduit and the user interface comprise complementary connectors that permit direct connection of the heated inspiratory conduit to the user interface following removal of the medical extension conduit.

6. The respiratory therapy assembly of claim 5, wherein the first end connector is substantially identical to a connector provided on the user interface.

7. The respiratory therapy assembly of any preceding claim, wherein the connector arrangement comprises:
at least one locking finger provided on one of the first end connector and a connector of the heated inspiratory conduit; and
at least one locking tab provided on the other of the first end connector and the connector of the heated inspiratory conduit, configured to engage a recess in the locking finger.

8. The respiratory therapy assembly of claim 7, wherein the locking finger comprises a chamfered tip configured to facilitate engagement with the locking tab.

9. The respiratory therapy assembly of claim 7 or claim 8, wherein two locking fingers are provided.

10. The respiratory therapy assembly of any one of claims 7 to 9, wherein the connector arrangement further comprises at least one alignment feature configured to prevent connection unless the first end connector and the connector of the heated inspiratory conduit are rotationally aligned.

11. The respiratory therapy assembly of claim 10, wherein the alignment feature comprises one or more recesses formed in a terminal face of first end connector and the connector of the heated inspiratory conduit.

12. The respiratory therapy assembly of any preceding claim, wherein the clip member comprises:
an annular body engageable with a surface relief feature of the heated inspiratory conduit and/or the medical extension conduit; and
a pair of jaws configured to grip a structure associated with the incubator.

13. The respiratory therapy assembly of claim 12, wherein the annular body is movable along the heated inspiratory conduit and/or the medical extension conduit in a first orientation and resistant to movement along the heated inspiratory conduit and/or the medical extension conduit in a second orientation.

14. The respiratory therapy assembly of claim 12 or claim 13, wherein the jaws are configured to be biased toward a closed position.

15. The respiratory therapy assembly of any preceding claim, further comprising a temperature sensor configured to be positioned outside the incubator and configured to provide a signal for controlling the heat delivered to the breathable gases by the heated inspiratory conduit.
